# EUROPEAN PATENT APPLICATION

(11) **EP 4 671 356 A1**
(43) Date of publication of application: **31.12.2025**
(21) Application number: 24760449.9
(22) Date of filing: 22.02.2024
(51) Int. Cl.: C12M 3/00

(54) **STRUCTURE AND CONTAINER**

(30) Priority: 24.02.2023 JP 2023026893; 24.02.2023 JP 2023026897
(71) Applicant: Mitsui Chemicals, Inc., Tokyo 104-0028 (JP)
(72) Inventor: SAKAJIRI, Aya, Chiba 299-0265 (JP); ESASHIKA, Katsuhiro, Chiba 299-0265 (JP)
(74) Representative: J A Kemp LLP
(86) International application number: PCT/JP2024/006641
(87) International publication number: WO 2024/177151

(57) **Abstract**

This structure constitutes a culture vessel having a plurality of accommodation sections in which cells are cultured. The structure comprises: a base that includes a plurality of through holes opening in the vertical direction; and an adherence layer which is provided on the bottom surface of the base and onto the bottom surface of which a sheet for closing the through holes is adhered. The adherence layer has openings that are located below the through holes and that have an outline greater than an outline of the through holes in plan view.

## Description

### Technical Field

The present invention relates to a structural body constituting a vessel and the vessel.

### Background Art

Conventionally, as disclosed in PTL 1, cell culture has been conducted using a culture vessel including compartments for culturing cells.

In addition, as a cell culture vessel, there are known configurations where a transparent resin sheet (or film) is attached to the bottom surface of a cylindrical base member to form a vessel, or where a transparent resin sheet is attached to the upper surface of a well to form a sealed vessel. Vessels configured by attaching a plurality of base boards to form a channel-shaped structure where liquid circulates or a well-shaped structure where liquid does not circulate are also known.

In manufacturing these vessels, adhesives or pressure-sensitive adhesives are used to attach transparent resin sheets or base boards. For example, PTL 2 describes that polydimethylsiloxane (PDMS) can be used as a pressure-sensitive adhesive for attaching a plurality of base boards.

In addition, as described in PTL 3, to observe substances such as proteins produced by cultured cells or to check the state of the cultured cells, agents such as fluorescent dyes capable of detecting biologically-derived substances may be introduced into the culture medium within the vessel.

### Citation List

### Patent Literature

PTL 1
   Japanese Patent Application Laid-Open No. 2022-149254
PTL 2
   WO2007/077607
PTL 3
   Japanese Patent Application Laid-Open No. 2014-093979

### Summary of Invention

### Technical Problem

Regarding the culture vessel disclosed in PTL 1, there is a demand for culture vessels with high culture functionality in recent years.

In view of such circumstances, an object of the present invention is, in its first aspect, to provide a structural body constituting a culture vessel with high culture functionality and the culture vessel.

Furthermore, regarding the culture vessel disclosed in PTL 2, pressure-sensitive adhesives that do not require curing are simpler for manufacturing cell culture vessels than adhesives that require curing after application and attachment. According to the inventors' findings, adhesives may shrink during curing, which can cause the attached transparent resin sheet to warp. When the transparent resin sheet warps, the observability inside the vessel decreases, reducing the observation efficiency of biologically-derived substances by the agent. In addition, the shrunken adhesive itself may lose transparency, further reducing the observation efficiency of biologically-derived substances.

In contrast, using a pressure-sensitive adhesive that does not require curing prevents the decrease in observation efficiency due to shrinkage during curing. However, according to the inventors' findings, when cells are cultured in a vessel using a pressure-sensitive adhesive, the observation efficiency may not increase as expected, for example, due to the fluorescence intensity of the fluorescent dye decreasing over time.

In view of such circumstances, another object of the present invention is, in its second aspect, to provide a structural body for manufacturing a vessel in which different members are attached with a pressure-sensitive adhesive, resulting in a vessel with improved observation efficiency of biologically-derived substances, and to provide the vessel manufactured from the structural body.

### Solution to Problem

A mode of the structural body related to the first aspect of the present invention is as follows. A structural body constituting a culture vessel that includes a plurality of compartments for culturing cells, the structural body including:
a base member including a plurality of through holes each opening in a vertical direction; and
an attachment layer provided on a lower surface of the base member, the attachment layer attaching a sheet for closing the through hole to the lower surface, in which
the attachment layer includes, under the through hole, an opening whose outer shape is larger than an outer shape of the through hole in plan view.

A mode of the vessel related to the first aspect of the present invention includes: the above-described structural body; and
a sheet closing the through hole and, together with the through hole, forming a compartment.

A mode of the structural body related to the second aspect of the present invention is as follows. A structural body, including:
a base member including a through hole or a recess; and
a pressure-sensitive adhesive layer provided on one surface of the base member, in which
the structural body forms a vessel by adhering another base member or a sheet via the pressure-sensitive adhesive layer to close at least one surface of the through hole or close the recess, the vessel allowing observation of a biologically-derived substance,
a pressure-sensitive adhesive forming the pressure-sensitive adhesive layer has features below
   a residual rate of Rhodamine B in a solution is 20% or more, the solution being obtained by affixing a test piece of the pressure-sensitive adhesive cut to a size of 10 mm × 10 mm to a bottom surface of a tissue culture-treated polystyrene well having an opening diameter of 16.2 mm, a well depth of 18 mm, and a well capacity of 3.5 mL, filling the well with 0.5 mL of a phosphate-buffered saline solution of the Rhodamine B at a concentration of 5 µM, and allowing the phosphate-buffered saline solution to stand at 23°C for 48 hours, and
   a generated load when a dynamic strain of 0.05% is applied to the test piece of the pressure-sensitive adhesive by using a solid viscoelasticity measuring device is 20g or less, the test piece having a width of 5 mm.

A mode of the vessel related to the second aspect of the present invention includes:
the structural body related to the second aspect of the present invention as described above; and
another base member or a sheet attached to the structural body via the pressure-sensitive adhesive layer.

### Advantageous Effects of Invention

According to the first aspect of the present invention, a culture vessel with high culture functionality can be provided.

Furthermore, according to the second aspect of the present invention, a structural body for manufacturing a vessel in which different members are attached with a pressure-sensitive adhesive, resulting in a vessel with improved observation efficiency of biologically-derived substances, and the vessel manufactured from the structural body are provided.

### Brief Description of Drawings

FIG. 1 is a perspective view of a culture vessel according to Embodiment 1;
FIG. 2 is a cross-sectional view of a part of the culture vessel;
FIG. 3 is a plan view of an adhesive layer;
FIG. 4 is a diagram for explaining the shapes of a through hole of a cylindrical portion and an opening of an attachment layer;
FIG. 5 is a diagram for explaining the amount of displacement between the through hole of the cylindrical portion and the opening of the attachment layer;
FIG. 6 is a diagram for explaining the amount of displacement between the through hole of the cylindrical portion and the opening of the attachment layer;
FIG. 7 is a cross-sectional view of a part of an adhesive layer;
FIG. 8 is a plan view of the adhesive layer according to an example of a variation;
FIG. 9 is a perspective view of a culture vessel according to Embodiment 2;
FIG. 10 is a cross-sectional view of a part of the culture vessel;
FIG. 11 is a plan view of the adhesive layer; and
FIG. 12 is a cross-sectional view of a part of the culture vessel according to Embodiment 3.

### Description of Embodiments

Hereinafter, the structural body and culture vessel according to the present invention will be described in detail with reference to the drawings. The structural body and culture vessel described below are examples of the structural body and culture vessel according to the present invention, and the present invention is not limited to the embodiments described below.

### [First Embodiment]

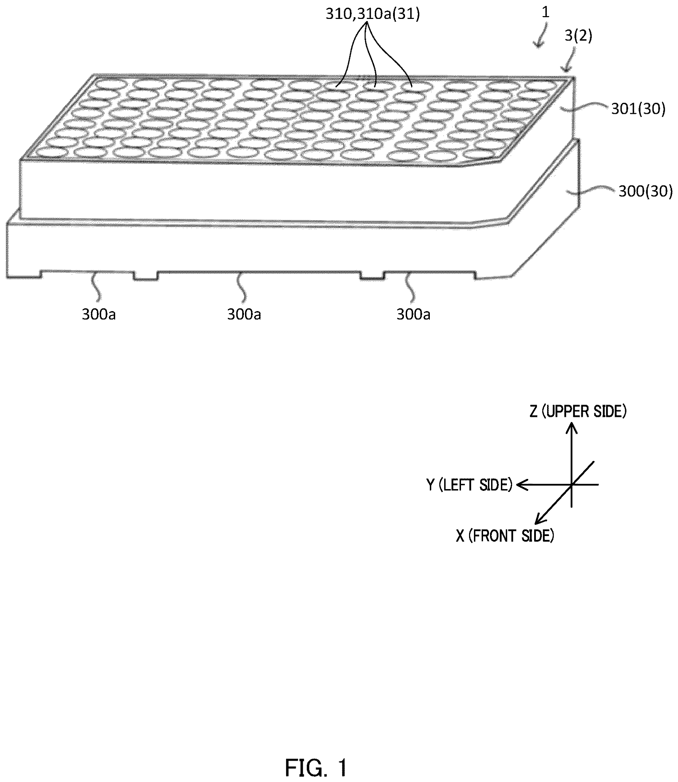

With reference to FIGS. 1 to 7, the structural body and culture vessel according to Embodiment 1 of the present invention will be described.

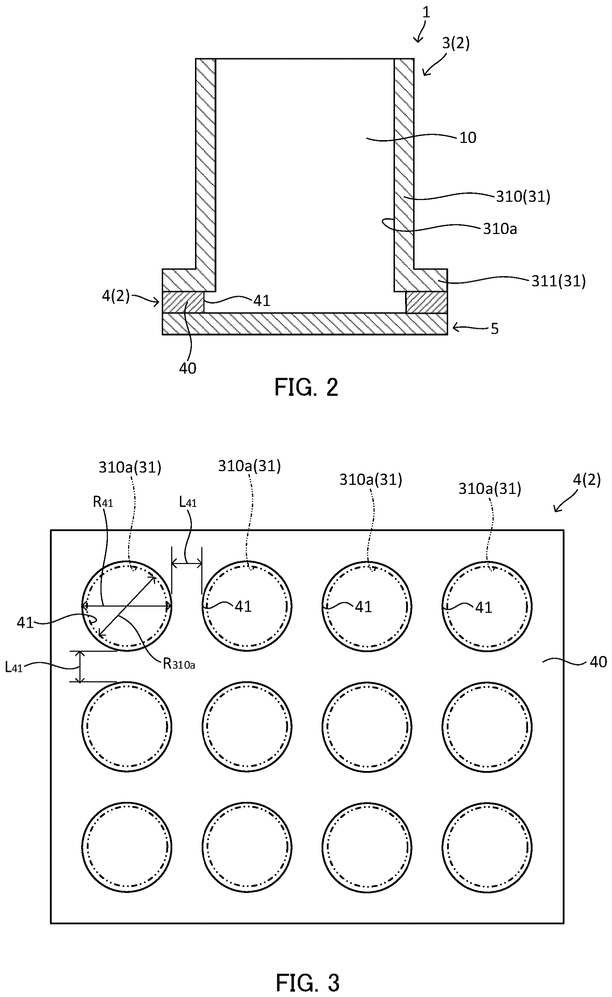

FIG. 1 is a perspective view of culture vessel 1 according to Embodiment 1 of the present invention. FIG. 2 is a cross-sectional view of a part of culture vessel 1.

Culture vessel 1 is used, for example, for culturing cells derived from humans. Such culture vessel 1 includes a plurality of compartments 10 (see FIG. 2) for culturing cells. Culture vessel 1 is housed in the culture space of a culture apparatus (e.g., an incubator) with compartment 10 containing an agent (culture medium) and cells to be cultured (hereinafter referred to as "target cells").

Culture vessel 1 of the present embodiment can be suitably used, for example, for culturing spheroids (cell aggregates) of target cells. Culture vessel 1 does not need to be used in a state housed in a culture apparatus. Culture vessel 1 may be used in various situations depending on the target cells.

Hereinafter, the specific configuration of culture vessel 1 will be described.

Culture vessel 1 includes structural body 2 and gas-permeable sheet 5.

In explaining the structure of culture vessel 1, the orthogonal coordinate system (X, Y, Z) illustrated in FIG. 1 may be used. The X direction coincides with the front-rear direction of culture vessel 1. The +side in the X direction coincides with the front side of culture vessel 1. The -side in the X direction coincides with the rear side of culture vessel 1. The Y direction coincides with the left-right direction of culture vessel 1. The +side in the Y direction coincides with the left side of culture vessel 1. The -side in the Y direction coincides with the right side of culture vessel 1. The Z direction coincides with the up-down direction (vertical direction) of culture vessel 1. The +side in the Z direction coincides with the upper side of culture vessel 1. The -side in the Z direction coincides with the lower side of culture vessel 1. The left, right, front, and rear sides of culture vessel 1 vary depending on the viewing direction of culture vessel 1 and are not limited to the left, right, front, and rear sides of culture vessel 1 illustrated in FIG. 1.

Structural body 2 includes main body 3 and adhesive layer 4.

Main body 3 corresponds to an example of a base member and includes frame 30 and main body-side compartment 31. Main body 3 is made of a synthetic resin, for example, and is an integrally molded product manufactured by injection molding.

Examples of the synthetic resin constituting main body 3 include polystyrene and polyolefin. The polyolefin may be a cyclic olefin (co)polymer, a 4-methyl-1-pentene (co)polymer, polypropylene, or polyethylene.

From the viewpoint of gas permeability, a 4-methyl-1-pentene (co)polymer is preferred as the synthetic resin constituting main body 3. From the viewpoint of heat resistance, a 4-methyl-1-pentene (co)polymer, polystyrene, polypropylene, or cyclic olefin (co)polymer is preferred as the synthetic resin constituting main body 3.

The instantaneous value of the Shore D hardness of main body 3 is, for example, 60 to 90. The instantaneous value of Shore D hardness is a value obtained by contacting a push needle with a part of main body 3 using a durometer hardness tester (D type) and immediately reading the scale (in accordance with ASTM D2240).

When the instantaneous value of the Shore D hardness is in the range of 60 to 90, gas-permeable sheet 5 described below can be attached to main body 3 with a satisfactory appearance.

Frame 30 is composed of a rectangular frame-shaped member. Frame 30 constitutes the outer shape of culture vessel 1.

Frame 30 includes lower frame 300 and upper frame 301.

Lower frame 300 is a rectangular frame-shaped member constituting the lower half of frame 30. Lower frame 300 corresponds to an example of a lower peripheral wall portion.

During the usage state of culture vessel 1, lower frame 300 is a part that is placed on a placement section provided in a culture apparatus (not illustrated), for example. Lower frame 300 has a height such that the lower surface of gas-permeable sheet 5 described below does not contact the placement surface of the placement section.

Lower frame 300 is provided to surround the side of the space under main body-side compartment 31 described below over the entire circumference of the space. The space under main body-side compartment 31 is also the space under gas-permeable sheet 5 described below (hereinafter referred to as "the space under gas-permeable sheet 5").

Lower frame 300 may include notches 300a at a plurality of locations on the lower end. During the usage state of culture vessel 1, notch 300a allows the space on the inner peripheral surface side of lower frame 300 (i.e., the space under gas-permeable sheet 5) to communicate with the space on the outer peripheral surface side of lower frame 300 (i.e., the external space).

Therefore, during the usage state of culture vessel 1, air from the external space is supplied to a region under gas-permeable sheet 5 through notches 300a.

As described above, the configuration in which lower frame 300 includes notches 300a contributes to the improvement of oxygen supply to the region under gas-permeable sheet 5. In addition, the oxygen supplied to the region under gas-permeable sheet 5 is delivered to the target cells housed in compartment 10 of culture vessel 1 through gas-permeable sheet 5, thereby promoting the culture of the target cells. Furthermore, the positions and/or number of the notches are not limited to the positions and/or number of the above-described notches 300a. Moreover, the notch may be omitted.

Upper frame 301 is a rectangular frame-shaped member that constitutes the upper half of frame 30. Upper frame 301 is provided on the upper side of lower frame 300. Upper frame 301 corresponds to an example of the upper peripheral wall portion.

Upper frame 301 is provided so as to surround main body-side compartment 31 over the entire circumference of the main body-side compartment.

The upper end of upper frame 301 has a shape that fits with the lower end of lower frame 300 of another culture vessel 1. In other words, culture vessel 1 is configured to allow a plurality of culture vessels 1 to be stacked vertically.

By fitting upper frame 301 of culture vessel 1 placed below with lower frame 300 of culture vessel 1 placed above, culture vessel 1 placed below and culture vessel 1 placed above can be stacked without wobbling.

Main body-side compartment 31 is provided in the space enclosed by frame 30. Main body-side compartment 31 is provided integrally with frame 30.

Main body-side compartment 31 includes a plurality of cylindrical portions 310 and bottom plate portion 311.

Cylindrical portion 310 is cylindrical and opens in the vertical direction. In the present embodiment, the shape of the opening of cylindrical portion 310 (in other words, the outer shape in plan view) is circular. Cylindrical portions 310 are arranged in the left-right direction and the front-rear direction.

The number of cylindrical portions 310 matches the number of compartments 10 for culturing cells. The number of cylindrical portions 310 may be, for example, 6, 24, 96, or 384. Of course, the number of cylindrical portions 310 may be other than 6, 24, 96, and 384.

Adjacent cylindrical portions 310 are connected by a connecting portion (not illustrated). In addition, the left end cylindrical portion 310, the right end cylindrical portion 310, the front end cylindrical portion 310, and the rear end cylindrical portion 310 are connected to the inner peripheral surface of frame 30 via a connecting portion (not illustrated).

The space defined by the inner peripheral surface of cylindrical portion 310 (hereinafter referred to as the "inner space of cylindrical portion 310") corresponds to an example of a through hole in the base member. The inner space of cylindrical portion 310, together with gas-permeable sheet 5 described below, constitutes compartment 10 for culturing cells. Hereinafter, the inner space of cylindrical portion 310 may simply be referred to as through hole 310a.

In the present embodiment, the outer shape of through hole 310a in plan view is circular. In other words, the inner peripheral surface of through hole 310a is cylindrical.

Plan view means viewing culture vessel 1 and structural body 2 from above (Z direction + side). Hereinafter, unless otherwise specified, when referring to the outer shape of through hole 310a, it means the outer shape of through hole 310a in plan view.

The outer shape of through hole 310a is preferably circular or square. From the viewpoint of improving the adhesion strength between gas-permeable sheet 5 and the periphery of through hole 310a, the outer shape of through hole 310a is more preferably circular. The reason is that when the outer shape of through hole 310a is circular, it is possible to attach more evenly at an attachment portion between the periphery of through hole 310a and gas-permeable sheet 5.

In addition, from the viewpoint of expanding the occupied volume of through hole 310a (in other words, the volume of compartment 10), the outer shape of through hole 310a is preferably square. For example, when the number of through holes 310a is large (e.g., 384), with the outer shape of through hole 310a being circular, the volume of compartment 10 may become small. In this case, when the outer shape of through hole 310a is a polygon such as a square, it becomes easier to increase the volume of compartment 10 than when the outer shape of through hole 310a is circular.

Bottom plate portion 311 is a plate-shaped member that connects the lower ends of cylindrical portions 310 to each other, as illustrated in FIG. 2. The outer edge of bottom plate portion 311 is connected to the inner peripheral surface of frame 30 over the entire circumference. The lower surface of bottom plate portion 311 is also the lower surface of main body-side compartment 31. In addition, the lower surface of bottom plate portion 311 is also the lower surface of main body 3. Therefore, the lower surface of bottom plate portion 311 corresponds to an example of the lower surface of the base member.

Hereinafter, adhesive layer 4 will be described with reference to FIGS. 2 to 7. Adhesive layer 4 corresponds to an example of an attachment layer and is provided on the lower surface of main body 3. Specifically, adhesive layer 4 is provided on the lower surface of main body-side compartment 31. Such adhesive layer 4 is made of a pressure-sensitive adhesive. Adhesive layer 4 is a member for attaching gas-permeable sheet 5 described below to the lower surface of main body-side compartment 31.

Adhesive layer 4 includes adhesive layer main body 40 and a plurality of openings 41.

Adhesive layer main body 40 is a sheet-shaped member that is to be attached to the lower surface of main body-side compartment 31 (specifically, bottom plate portion 311) and gas-permeable sheet 5.

Opening 41 is provided in adhesive layer main body 40. Opening 41 extends through adhesive layer main body 40 in the vertical direction.

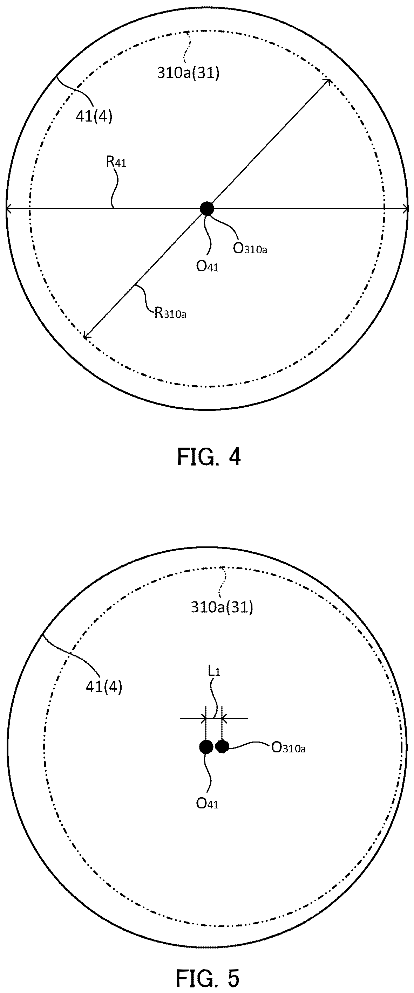

In the state where the adhesive layer main body is attached to the lower surface of main body-side compartment 31, opening 41 is disposed under through hole 310a of main body-side compartment 31, as illustrated in FIG. 2. As illustrated in FIGS. 2 to 4, the outer shape of opening 41 is larger than the outer shape of through hole 310a in plan view. In FIGS. 3 and 4, opening 41 is illustrated by a solid line. In FIGS. 3 and 4, through hole 310a is illustrated by a two-dot chain line.

In the present embodiment, the outer shape of opening 41 in plan view is circular, as illustrated in FIGS. 3 and 4. Hereinafter, when referring to the outer shape of opening 41, it means the outer shape of opening 41 in plan view. In addition, the outer shape of through hole 310a is also circular. The outer shape of opening 41 is similar to the outer shape of through hole 310a.

In FIG. 4, center O₄₁ of the outer shape of opening 41 coincides with center O₃₁₀ₐ of the outer shape of through hole 310a. In this state, the peripheral portion of opening 41 does not overlap with through hole 310a in plan view.

When the peripheral portion of opening 41 overlaps with through hole 310a in plan view, the contact area between adhesive layer 4 and an agent contained in compartment 10 may increase during the usage state of culture vessel 1. When the contact area between adhesive layer 4 and the agent contained in compartment 10 increases, the amount of the agent adsorbed by adhesive layer 4 increases, potentially reducing the concentration of the agent in compartment 10. A decrease in the concentration of the agent in compartment 10 is undesirable as it can lead to a decline in culture functionality.

On the other hand, in the present embodiment, the peripheral portion of opening 41 does not overlap with through hole 310a in plan view. Therefore, the contact area between adhesive layer 4 and the agent is small during the usage state of culture vessel 1. In other words, the amount of the agent adsorbed by adhesive layer 4 is small. As a result, the decline in culture functionality is prevented. Hereinafter, the effect achieved by such culture vessel 1 of the present embodiment is referred to as the agent adsorption preventing effect of culture vessel 1.

The positional relationship between opening 41 and through hole 310a is most preferably the positional relationship between opening 41 and through hole 310a illustrated in FIGS. 3 and 4.

However, as illustrated in FIG. 5, as long as the peripheral portion of opening 41 does not overlap with through hole 310a, center O₄₁ of the outer shape of opening 41 and center O₃₁₀ₐ of through hole 310a may be misaligned.

When the positional relationship between the peripheral portion of opening 41 and through hole 310a is the positional relationship between the peripheral portion of opening 41 and through hole 310a illustrated in FIG. 5, the above-described agent adsorption preventing effect can be obtained.

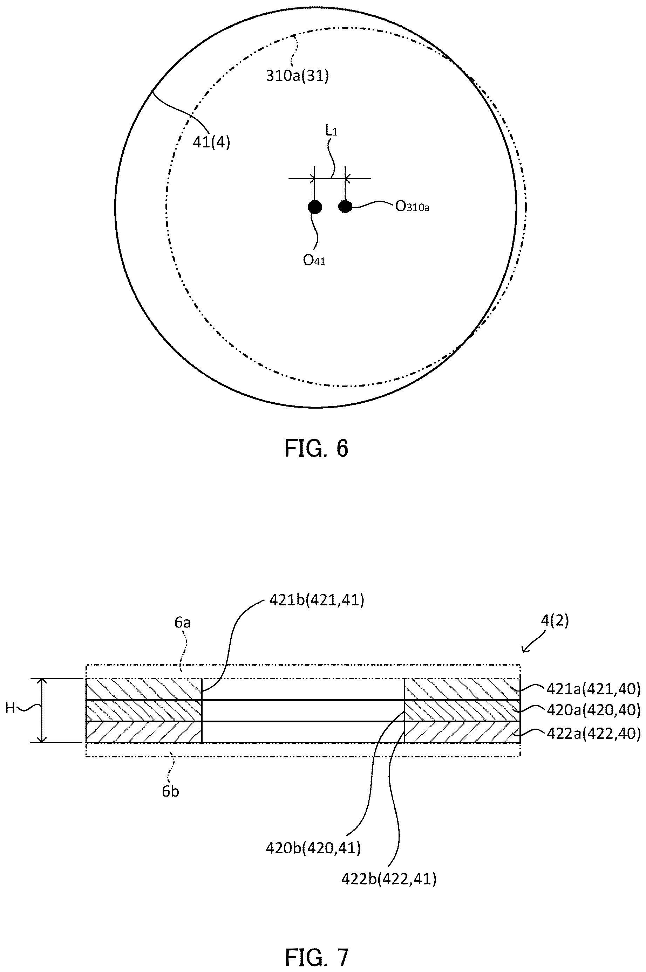

In addition, as illustrated in FIG. 6, even with a configuration such that a part of the peripheral portion of opening 41 overlaps with through hole 310a, it is possible to obtain part of the above-described agent adsorption preventing effect.

The difference between radius R₄₁ of opening 41 and radius R₃₁₀ₐ of through hole 310a may be smaller than half the distance L₄₁ between the ends of adjacent openings 41. In the present embodiment, the distance between the ends of openings 41 adjacent to each other in the vertical direction in FIG. 3 is equal to the distance between the ends of openings 41 adjacent to each other in the left-right direction in FIG. 3.

When the distance between the ends of adjacent openings 41 differs depending on the direction in which the openings are adjacent to each other, the smallest distance becomes the distance between the ends of adjacent openings 41.

In addition, distance L1 between center O₄₁ of opening 41 and center O₃₁₀ₐ of through hole 310a may be equal to or smaller than half the difference between radius R₄₁ of opening 41 and radius R₃₁₀ₐ of through hole 310a. The distance between center O₄₁ of opening 41 and center O₃₁₀ₐ of through hole 310a is also the amount of displacement between center O₄₁ of opening 41 and center O₃₁₀ₐ of through hole 310a.

Adhesive layer 4 has a laminated structure in which a plurality of sheet-shaped members are stacked. Specifically, as illustrated in FIG. 7, adhesive layer 4 includes base layer 420, upper adhesive layer 421, and lower adhesive layer 422. The thickness dimension H of adhesive layer 4 may satisfy, for example, 20 µm ≤ H ≤ 150 µm.

FIG. 7 schematically illustrates a partial cross-cross-sectional view of adhesive layer 4. In addition, in FIG. 7, upper protective film 6a provided on the upper surface of upper adhesive layer 421 and lower protective film 6b provided on the lower surface of lower adhesive layer 422 are illustrated by two-dot chain lines.

Adhesive layer 4 constitutes culture vessel 1 with upper protective film 6a and lower protective film 6b removed. In addition, adhesive layer 4 constitutes structural body 2 with only upper protective film 6a of upper protective film 6a and lower protective film 6b removed.

Base layer 420 is a sheet-shaped member made of a resin. The resin constituting base layer 420 may be, for example, polyethylene terephthalate (PET).

Base layer 420 includes base layer side main body 420a and base layer side opening 420b.

Base layer side main body 420a is a sheet-shaped member made of a resin. Upper adhesive layer 421 is attached to the upper surface of base layer side main body 420a. Lower adhesive layer 422 is attached to the lower surface of base layer side main body 420a. Base layer side main body 420a constitutes the intermediate layer of adhesive layer main body 40.

Base layer side opening 420b is provided in base layer side main body 420a. Base layer side opening 420b extends through base layer side main body 420a in the vertical direction. Base layer side opening 420b constitutes the intermediate portion of opening 41.

Upper adhesive layer 421 is an example of a first adhesive layer and is a sheet-shaped member with tackiness.

Upper adhesive layer 421 includes upper layer side main body 421a and upper layer side opening 421b.

Upper layer side main body 421a is a sheet-shaped member made of a pressure-sensitive adhesive. Upper layer side main body 421a is attached to the upper surface of base layer side main body 420a. The pressure-sensitive adhesive constituting upper layer side main body 421a may be, for example, an acrylic or silicone pressure-sensitive adhesive. Upper layer side main body 421a constitutes the upper portion of adhesive layer main body 40.

Upper layer side opening 421b is provided in upper layer side main body 421a. Upper layer side opening 421b extends through upper layer side main body 421a in the vertical direction. The inner diameter of upper layer side opening 421b is equal to the inner diameter of base layer side opening 420b.

In the state where upper adhesive layer 421 is attached to the upper surface of base layer side main body 420a, the central axis of base layer side opening 420b coincides with the central axis of upper layer side opening 421b. Upper layer side opening 421b constitutes the upper end of opening 41.

Lower adhesive layer 422 is an example of a second adhesive layer and is a sheet-shaped member with tackiness.

Lower adhesive layer 422 includes lower layer side main body 422a and lower layer side opening 422b.

Lower layer side main body 422a is a sheet-shaped member made of a pressure-sensitive adhesive. Lower layer side main body 422a is attached to the lower surface of base layer side main body 420a. Lower layer side main body 422a constitutes the lower portion of adhesive layer main body 40.

The pressure-sensitive adhesive constituting lower layer side main body 422a is the same as the pressure-sensitive adhesive constituting upper layer side main body 421a. However, the pressure-sensitive adhesive constituting lower layer side main body 422a may be different from the pressure-sensitive adhesive constituting upper layer side main body 421a.

Lower layer side opening 422b is provided in lower layer side main body 422a. Lower layer side opening 422b extends through lower layer side main body 422a in the vertical direction. The inner diameter of lower layer side opening 422b is equal to the inner diameter of base layer side opening 420b.

In the state where lower adhesive layer 422 is attached to the lower surface of base layer side main body 420a, the central axis of base layer side opening 420b coincides with the central axis of lower layer side opening 422b. Lower layer side opening 422b constitutes the lower end of opening 41.

Although not illustrated, the adhesive layer may have a single-layer structure. In this case, the adhesive layer may be constituted by a base layer made of nonwoven fabric and a pressure-sensitive adhesive composition adhering to or saturating the base layer.

Furthermore, the outer shape of the opening in the adhesive layer is not limited to circular. The outer shape of the opening may be, for example, circular, rectangular, or regular polygonal.

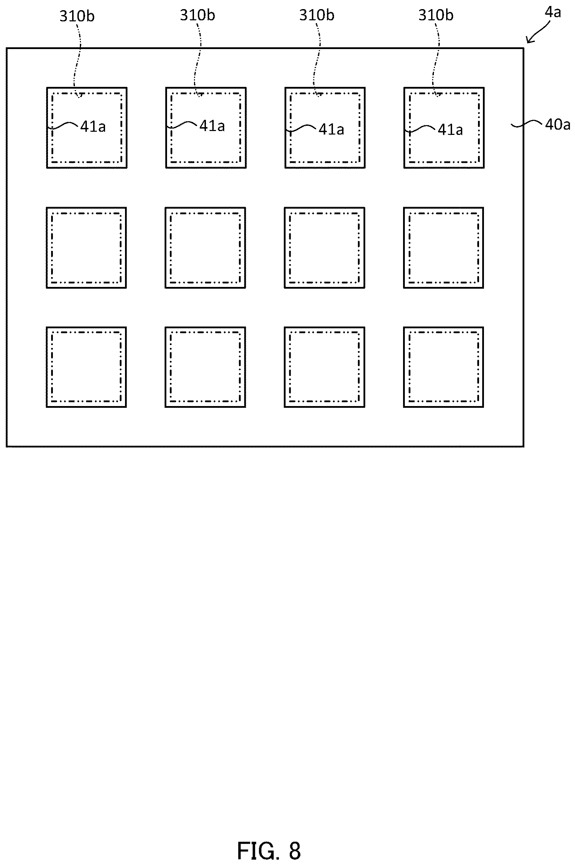

FIG. 8 is a diagram showing an example of a variation of the adhesive layer. Adhesive layer 4a illustrated in FIG. 8 includes adhesive layer main body 40a and a plurality of openings 41a.

In this example, the outer shape of opening 41a is square. As illustrated in FIG. 8, the outer shape of opening 41a is larger than the shape of through hole 310b in plan view. FIG. 8 is illustrated in a similar manner to FIG. 3. In FIG. 8, opening 41a is illustrated by a solid line. In addition, in FIG. 8, through hole 310b is illustrated by a two-dot chain line. The other configurations of adhesive layer 4a are substantially the same as the configuration of adhesive layer 4 in Embodiment 1 described above.

The attachment layer is not limited to adhesive layer 4 described above. For example, the attachment layer may be constituted by a layer containing an adhesive.

Gas-permeable sheet 5 is a flat sheet-shaped member. Gas-permeable sheet 5 is attached to the lower surface of main body 3 via adhesive layer 4. Specifically, gas-permeable sheet 5 is attached to the lower surface of bottom plate portion 311 in main body-side compartment 31 via adhesive layer 4.

Gas-permeable sheet 5 is a sheet-shaped member with an oxygen permeability of 200 to 60,000 cm³/ (m² × 24hr × atm). Gas-permeable sheet 5 is, for example, a sheet-shaped member containing a polymer having structural units derived from 4-methyl-1-pentene.

The above oxygen permeability is a value [cm³/ (m² × 24hr × atm)] measured using a differential pressure gas permeability measuring device MT-C3 manufactured by Toyo Seiki Seisaku-sho, Ltd., at a temperature of 23°C and a humidity of 0%. The measurement sample is prepared by cutting a 90 × 90 mm test piece from gas-permeable sheet 5. The measurement section diameter is set to 70 mm (with a permeation area of 38.46 cm²). When a high oxygen permeability is expected, it is more preferable to apply an aluminum mask to the measurement sample in advance to set the actual permeation area to 5 cm².

Gas-permeable sheet 5 closes the lower opening of through hole 310a in main body-side compartment 31. The upper surface of gas-permeable sheet 5, together with through hole 310a, constitutes compartment 10 of culture vessel 1.

Hereinafter, a method for culturing spheroids using culture vessel 1 of the present embodiment described above will now be briefly described. An operator places an agent (culture medium) and target cells into compartment 10 of culture vessel 1. The target cells may be, for example, cells collected from a human. The culture medium may be appropriately selected according to the target cells. The target cells settle at the lower end of compartment 10 (i.e., the upper surface of gas-permeable sheet 5).

Next, the operator places culture vessel 1 into the culture space of a culture apparatus (not illustrated) such as an incubator. The environmental conditions of the culture space are set to be suitable for culturing the target cells. After a predetermined time has elapsed, the cells are cultured within compartment 10 of culture vessel 1.

In culture vessel 1 of the present embodiment, the lower region of compartment 10 is constituted by gas-permeable sheet 5. Since gas-permeable sheet 5 has excellent oxygen permeability, a large amount of oxygen is supplied to the target cells that have settled at the lower end of compartment 10. Therefore, the culture of the target cells is promoted.

In addition, cultured cells tend to gather at the lower end of compartment 10. Therefore, spheroids (cell aggregates) of the target cells are easily formed at the lower end of compartment 10. As described above, culture vessel 1 of the present embodiment can be suitably used for culturing spheroids (cell aggregates) of the target cells.

### (Effects and Advantages of the Present Embodiment)

According to culture vessel 1 of the present embodiment having the above configuration, a culture vessel with high culture functionality can be provided. The reasons for this will be explained below.

First, in the case of culture vessel 1 according to the present embodiment, adhesive layer 4 includes opening 41 whose outer shape is larger than the outer shape of through hole 310a in plan view, as illustrated in FIGS. 3 and 4. Therefore, the peripheral portion of opening 41 is less likely to overlap with through hole 310a in plan view. Thus, the contact area between adhesive layer 4 and the agent during the usage state of culture vessel 1 is reduced, and the amount of the agent adsorbed by adhesive layer 4 can be reduced. As a result, the decrease in agent concentration can be prevented, thereby improving the culture functionality of culture vessel 1. In addition, as will be described below, culture vessel 1 may also contain a reagent for fluorescent observation of cultured cells. According to culture vessel 1 of the present embodiment, the amount of such a reagent adsorbed by adhesive layer 4 can be suppressed. As a result, culture vessel 1 of the present embodiment can achieve high observation efficiency.

Particularly, in the case of the present embodiment, as illustrated in FIGS. 3 and 4, the peripheral portion of opening 41 does not overlap with through hole 310a in plan view. Such a configuration can minimize the contact area between adhesive layer 4 and an agent. Therefore, the amount of an agent adsorbed by adhesive layer 4 can be significantly reduced. Other effects and advantages obtained from culture vessel 1 of the present embodiment are as described above.

### (Preferred Mode of Adhesive Layer 4)

In the present embodiment, adhesive layer 4 can satisfy one or more of the following conditions.

(Condition 1) The residual rate of Rhodamine B in the aqueous solution obtained by the following procedure is 20% or more: a test piece cut to a size of 10 mm × 10 mm is affixed to the bottom surface of a tissue culture-treated polystyrene (TCPS) well having an opening diameter of 16.2 mm, a well depth of 18 mm, and a well capacity of 3.5 mL; the well is filled with 0.5 mL of a phosphate-buffered saline (PBS) solution of Rhodamine B at a concentration of 10 µmol/L and left to stand at 23°C for 48 hours.

According to the findings of the inventors, adhesive layer 4 easily adsorbs agents, which may reduce the concentration of an agent introduced into compartment 10.

Based on the above findings, the inventors have confirmed the adsorption amounts of various agents for a plurality of pressure-sensitive adhesives and found that Rhodamine B can serve as an indicator for predicting the amounts of various agents adsorbed by pressure-sensitive adhesives. In other words, compared to other agents, Rhodamines are more likely to exhibit differences in adsorption amounts depending on the pressure-sensitive adhesive. Using a pressure-sensitive adhesive with a low adsorption amount of Rhodamine B allows for high observation efficiency even when other agents are used for the observation.

Condition 1 is based on these findings and specifies that adhesive layer 4 has a low adsorption amount of Rhodamine B. Adhesive layer 4 that satisfies condition 1 is less likely to adsorb various agents at an exposed site on the inner surface of compartment 10.

Regarding condition 1, in the present embodiment, adhesive layer 4 is cut into a size of 10 mm × 10 mm to create a test piece. The thickness of the test piece is the thickness of a purchased pressure-sensitive adhesive itself when it is a commercial product, and it is also the thickness of adhesive layer 4. Then, the test piece is affixed to the bottom surface of a tissue culture-treated polystyrene well having an opening diameter of 16.2 mm, a well depth of 18 mm, and a well capacity of 3.5 mL, the well is filled with 0.5 mL of a phosphate-buffered saline (PBS) solution of Rhodamine B at a concentration of 5 µM and left to stand at 23°C for 48 hours. When the residual rate of Rhodamine B in the aqueous solution after standing is 20% or more (i.e., the concentration of Rhodamine B in the aqueous solution after standing is 1 µM or more), adhesive layer 4 is considered to satisfy condition 1.

The amount of Rhodamine B in the aqueous solution after standing is measured by fluorescence analysis according to the following procedure.
Measuring device: FP-6600, manufactured by JASCO Corporation
Cell used: Quartz microcell
Bandwidth: Excitation side: 5nm, Fluorescence side: 6nm
Sensitivity (PMT voltage): 400V
Excitation wavelength: 555nm (Rhodamine B)
Fluorescence measurement wavelength: 580nm (Rhodamine B)
Scan speed: 2,000nm/min

From the above viewpoint, the residual rate of Rhodamine B in condition 1 is preferably 30% or more, more preferably 40% or more. Although the upper limit of the residual rate of Rhodamine B in condition 1 is not particularly limited, the upper limit can be 100%.

(Condition 2) To the test piece of adhesive layer 4 having a width of 5 mm, the generated load when a dynamic strain of 0.05% is applied by using a solid viscoelasticity measuring device is 20g or less.

Condition 2 means that adhesive layer 4 is easily deformable even with a smaller force. Such adhesive layer 4 easily conforms to the shape of the bottom surface (lower surface) of main body-side compartment 31 and is less likely to cause fine gaps or bends when attached. Therefore, adhesive layer 4 that satisfies condition 2 is less likely to cause a decrease in observation efficiency due to unintended refraction of light caused by these gaps or bends. In addition, adhesive layer 4 that satisfies condition 2 reduces the contact area with a liquid (culture medium or the like) filling the inside of compartment 10, thereby reducing the adsorption amount of various agents by adhesive layer 4.

Regarding condition 2, in the present embodiment, adhesive layer 4 is cut into a size of 5 mm width and 30 mm length to create a test piece. The thickness of the test piece is the thickness of adhesive layer 4. In the case of performing solid viscoelasticity measurement under the following conditions, when the generated load for applying a dynamic strain of 0.05% to the test piece under the following conditions of solid viscoelasticity measurement is 20g or less, adhesive layer 4 is considered to satisfy condition 2.
Measuring device: RSA-III, manufactured by TA Instruments Japan Inc.
Deformation mode: Tensile
Temperature: 23°C
Frequency: 1Hz
Strain: 0.05%
Atmospheric environment: Nitrogen atmosphere

From the above viewpoint, the generated load when a dynamic strain of 0.05% is applied in condition 2 is preferably 18g or less, more preferably 15g or less. Although the lower limit of the generated load when a dynamic strain of 0.05% is applied in condition 2 is not particularly limited, from the viewpoint of facilitating attachment, the lower limit can be 2g or more, preferably 5g or more.

### (Condition 3)

The glass transition temperature (Tg) is -122°C or higher.

Condition 3 means that the pressure-sensitive adhesive forming adhesive layer 4 does not have excessively high molecular mobility. In other words, when the mobility of the molecules that constitute a pressure-sensitive adhesive is high, the pressure-sensitive adhesive tends to adsorb agents more easily. In contrast, a pressure-sensitive adhesive that satisfies condition 3 has a moderately low mobility of the molecules that constitute the pressure-sensitive adhesive, making it less likely to adsorb various agents at an exposed site on the inner surface of compartment 10. Particularly, when the pressure-sensitive adhesive is an acrylic pressure-sensitive adhesive, the higher the Tg, the more likely the adsorption amount of agents decreases.

Regarding condition 3, in the present embodiment, the solid viscoelastic temperature dispersion measurement of a pressure-sensitive adhesive is performed under the following conditions, and the peak top value of the loss tangent (tanδ) obtained is used as the glass transition temperature (Tg) of the pressure-sensitive adhesive.

### (Measurement Conditions)

Measuring device: RSA-III, manufactured by TA Instruments Japan Inc.
Deformation mode: Tensile
Temperature range: -100°C to 25°C
Damping rate: 3°C/min
Frequency: 1Hz
Strain: 0.05%
Atmospheric environment: Air

For enhancing tackiness, a lower Tg of the pressure-sensitive adhesive is preferable. In contrast, from the viewpoint of improving the observation efficiency of biologically-derived substances based on the above findings, a higher Tg of the pressure-sensitive adhesive is preferable. From the viewpoint of balancing these, the Tg of the pressure-sensitive adhesive in condition 3 is more preferably -121°C to -30°C, even more preferably -80°C to -30°C, and particularly preferably -35°C to -30°C.

### (Condition 4)

It includes an acrylic pressure-sensitive adhesive having a structure derived from an aromatic ring-containing compound.

With an acrylic pressure-sensitive adhesive having a structure derived from an aromatic ring-containing compound, the aromatic ring (benzene ring) reduces molecular mobility. Therefore, the pressure-sensitive adhesive that satisfies condition 3 has a low mobility of the molecules that constitute the pressure-sensitive adhesive, and thus is less likely to adsorb various agents at an exposed site on the inner surface of compartment 10. The aromatic ring-containing compounds are not particularly limited as long as they contain an aromatic ring (benzene ring). Examples of the aromatic ring-containing compounds include aromatic vinyl compounds such as styrene, styrene sulfonic acid and the salts thereof, α-methylstyrene, p-t-butylstyrene, butoxystyrene, vinyltoluene, chlorostyrene, and vinylnaphthalene.

Regarding condition 3, in the present embodiment, the surface of adhesive layer 4 is measured by Fourier transform infrared spectroscopy (FT/IR) under the following conditions, and when a peak corresponding to the structure derived from styrene at 700 cm⁻¹ is confirmed in the obtained IR spectrum, adhesive layer 4 is considered to satisfy condition 4.

### (Measurement Conditions)

Measuring device: Nicolet iS50, manufactured by Thermo Fisher Scientific Measurement method: Total reflection measurement method using Ge prism (ATR method)
Resolution: 4 cm⁻¹
Number of integrations: 32 times

### (Condition 5)

It includes a silicone pressure-sensitive adhesive with the ratio ((Si-CH₃)/(Si-O)) of 0.500 or more and 0.600 or less between the peak intensity at 843 cm⁻¹ derived from Si-CH₃ and the peak intensity at 1068 cm⁻¹ derived from Si-O in the IR spectrum.

### (Condition 6)

It includes a silicone pressure-sensitive adhesive with the ratio ((Si-CH₃)/(Si-O)) of 0.280 or more and 0.300 or less between the peak intensity at 756 cm⁻¹ derived from Si-CH₃ and the peak intensity at 1068 cm⁻¹ derived from Si-O in the IR spectrum.

Conditions 5 and 6 specify the amount of terminal methyl groups relative to the siloxane bonds in a silicone pressure-sensitive adhesive, indicating that the molecular weight of the silicone pressure-sensitive adhesive is of an appropriate size. Pressure-sensitive adhesives that satisfy condition 5 or condition 6 have a moderately low mobility of the molecules that constitute the pressure-sensitive adhesive, and thus is less likely to adsorb various agents at an exposed site on the inner surface of compartment 10. On the other hand, in a pressure-sensitive adhesive that satisfy condition 5 or condition 6, the molecules that constitute the pressure-sensitive adhesive have sufficient mobility, and therefore the pressure-sensitive adhesive has sufficiently high tackiness.

A pressure-sensitive adhesive that satisfies condition 5 or condition 6 may satisfy either one or both of these conditions.

In addition, conditions 5 and 6 are the ratios of the respective peak intensities in the IR spectrum obtained under the same conditions as condition 3.

(Condition 7) The residual rate of Rhodamine 6G in the aqueous solution obtained by the following procedure is 20% or more: a test piece cut to a size of 10 mm × 10 mm is affixed to the bottom surface of a tissue culture-treated polystyrene well having an opening diameter of 16.2 mm, a well depth of 18 mm, and a well capacity of 3.5 mL, and the well is filled with 0.5 mL of a PBS solution of Rhodamine 6G at a concentration of 5 µM and left to stand at 23°C for 48 hours.

According to the findings of the inventors, in a similar manner to Rhodamine B in condition 1, Rhodamine 6G is also more likely to exhibit differences in adsorption amounts depending on the pressure-sensitive adhesive compared to other agents. A pressure-sensitive adhesive with a low adsorption amount of Rhodamine 6G tend to have a low adsorption amount for other agents as well. Therefore, adhesive layer 4 that satisfies condition 7 is less likely to adsorb various agents at an exposed site on the inner surface of compartment 10.

Condition 7 is the residual rate of Rhodamine 6G in the solution after standing, obtained under the same conditions as condition 1 except that a 5 µM Rhodamine 6G aqueous solution is used instead of a 0.5 mL/well Rhodamine B solution.

From the above viewpoint, the residual rate of Rhodamine 6G in condition 1 is preferably 30% or more, more preferably 40% or more, and even more preferably 50% or more. Although the upper limit of the residual rate of Rhodamine 6G in condition 7 is not particularly limited, the upper limit can be 100%.

(Condition 8) The weight average molecular weight (Mw) is 1,000 or more and 5,000,000 or less.

Condition 8 means that the molecular weight of the pressure-sensitive adhesive is of an appropriate size. A pressure-sensitive adhesive that satisfies condition 8 has a moderately low mobility of the molecules that constitute the pressure-sensitive adhesive, and thus is less likely to adsorb various agents on an exposed site on the inner surface of compartment 10. On the other hand, in a pressure-sensitive adhesive that satisfies condition 8, the molecules that constitute the pressure-sensitive adhesive have sufficient mobility, and therefore the pressure-sensitive adhesive has sufficiently high tackiness. From the above viewpoint, the weight average molecular weight (Mw) of the pressure-sensitive adhesive in condition 8 is preferably 2,000 or more and 2,000,000 or less, and more preferably 3,000 or more and 1,000,000 or less.

Condition 8 is the weight average molecular weight of a pressure-sensitive adhesive measured by gel permeation chromatography (GPC) under the following conditions.
Column: 2 × PLgel 5µ MIXED-C (7.5 mm × 300 mm), manufactured by Agilent Technologies
Column temperature: 40°C
Mobile phase: Tetrahydrofuran for HPLC (containing stabilizer), (manufactured by Fujifilm Wako Pure Chemical Corporation)
Flow rate: 1 mL/min
Injection volume: 100 µL
Detection: RI (Refractive Index)
Column calibration: Monodisperse polystyrene (EasiCal PS-1, manufactured by Agilent Technologies)
Molecular weight calibration: Relative calibration method (polystyrene equivalent) Device: 515 HPLC Pump/717plus (auto-injector)/Shodex RI-101 (differential refractive index detector), manufactured by Nihon Waters K.K.

(Condition 9) The storage modulus (E') is 1 × 10⁵ Pa or more and 1 × 10¹⁰ Pa or less.

(Condition 10) The loss tangent (tanδ), which is the ratio (E"/ E') of the storage modulus (E') to the loss modulus (E"), is 0.0100 or more and 1.0000 or less.

(Condition 11) The loss modulus (E") is 1 × 10⁶ Pa or more and 5 × 10⁸ Pa or less.

Regarding conditions 9, 10, and 11, in the present embodiment, the above values are obtained by the solid viscoelastic measurement performed under the same measurement conditions as condition 2.

### (Measurement Conditions)

Measuring device: RSA-III, manufactured by TA Instruments,
Deformation mode: Tensile
Temperature: 23°C
Frequency: 1Hz
Strain: 0.05%
Atmospheric environment: Nitrogen environment

The storage modulus (E') of adhesive layer 4 in condition 9 can be, for example, 1 × 10⁶ Pa or more, 1 × 10⁷ Pa or more, 1 × 10⁸ Pa or more, or 1 × 10⁹ Pa or more, and can also be 1 × 10⁹ Pa or less, 1 × 10⁸ Pa or less, 1 × 10⁷ Pa or less, or 1 × 10⁶ Pa or less.

The loss tangent (tanδ) of adhesive layer 4 in condition 10 can be, for example, 0.0500 or more, 0.1000 or more, 0.1500 or more, 0.2000 or more, 0.2500 or more, 0.3000 or more, 0.3500 or more, 0.4000 or more, 0.4500 or more, 0.5000 or more, 0.5500 or more, 0.6000 or more, 0.6500 or more, 0.7000 or more, 0.7500 or more, 0.8000 or more, 0.8500 or more, 0.9000 or more, or 0.9500 or more, and can also be 0.9500 or less, 0.9000 or less, 0.8500 or less, 0.8000 or less, 0.7500 or less, 0.7000 or less, 0.6500 or less, 0.6000 or less, 0.5500 or less, 0.5000 or less, 0.4500 or less, 0.4000 or less, 0.3500 or less, 0.3000 or less, 0.2500 or less, 0.2000 or less, 0.1500 or less, or 0.1000 or less.

The loss modulus (E") of a pressure-sensitive adhesive in condition 10 is preferably 1 × 10⁶ Pa or more and 5 × 10⁸ Pa or less, and more preferably 5 × 10⁶ Pa or more and 1 × 10⁸ Pa or less. When the loss modulus (E") of the pressure-sensitive adhesive is within this range, the viscosity of the pressure-sensitive adhesive is low, so that an agent is less likely to remain inside the pressure-sensitive adhesive, resulting in better agent adsorption.

(Condition 11) The peel strength of a pressure-sensitive adhesive layer at -50°C is 0.1 N/25 mm or more.

The peel strength of a pressure-sensitive adhesive layer in condition 11 can be, for example, 0.2 N/25 mm or more, 0.3 N/25 mm or more, 1 N/25 mm or more, 5 N/25 mm or more, 10 N/25 mm or more, 15 N/25 mm or more, or 20 N/25 mm or more.

When the peel strength of a pressure-sensitive adhesive layer at -50°C is equal to or more than the lower limit, the structural body and the base member or sheet are firmly bonded to each other even at a low temperature, preventing delamination due to impact, or the like. In other words, impact resistance at a low temperature can be improved.

The upper limit of the peel strength of the pressure-sensitive adhesive layer at - 50°C is not particularly limited and can be, for example, 200 N/25 mm or less.

### [Second Embodiment]

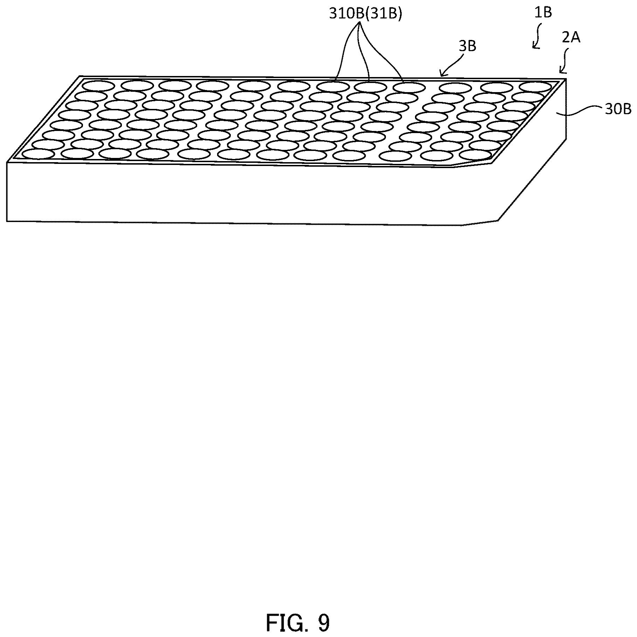

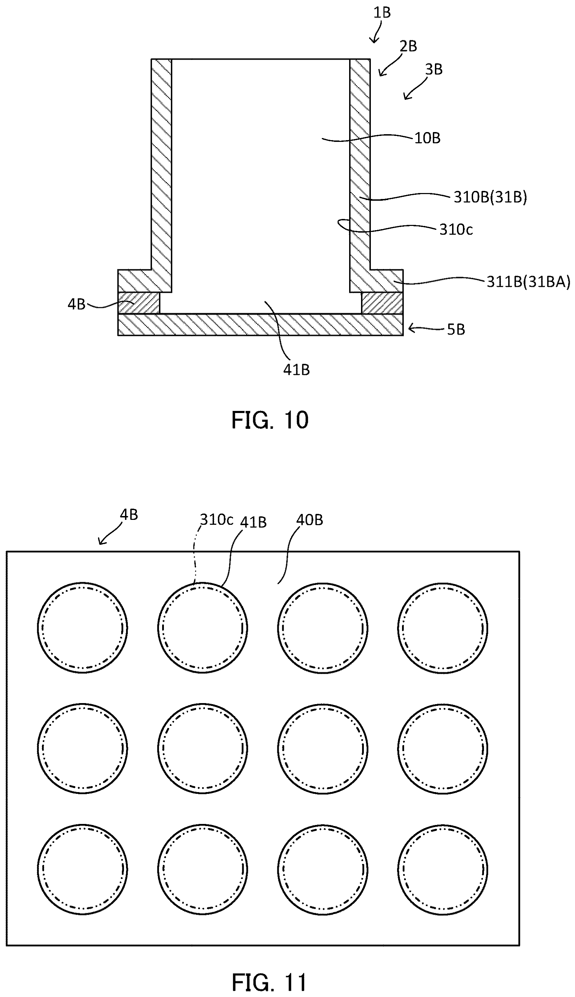

FIG. 9 is a perspective view of culture vessel 1B according to Embodiment 2 of the present invention. FIG. 10 is a partial cross-cross-sectional view of culture vessel 1B.

Culture vessel 1B is used, for example, for culturing cells derived from humans. Such culture vessel 1B has a plurality of compartments 10B (see FIG. 10) for culturing cells. Culture vessel 1B is housed in the culture space of a culture apparatus (e.g., an incubator) with compartment 10B containing an agent (culture medium) and cells to be cultured (hereinafter referred to as "target cells").

Culture vessel 1B of the present embodiment can be suitably used for culturing spheroids (cell aggregates) of target cells. Culture vessel 1B does not need to be used in a state housed in a culture apparatus. Culture vessel 1B may be used in various situations depending on the target cells.

Hereinafter, the specific configuration of culture vessel 1B will be described.

Culture vessel 1B includes structural body 2B and transparent resin sheet 5B.

Structural body 2B includes main body 3B and pressure-sensitive adhesive layer 4B.

Main body 3B includes frame 30B and main body-side compartment 31B. Main body 3B is made of synthetic resin, for example, and is an integrally molded product made by injection molding. Examples of the synthetic resin constituting main body 3B include polystyrene and polyolefins. Examples of the polyolefins include cyclic olefin (co)polymers, 4-methyl-1-pentene (co)polymers, polypropylene, and polyethylene. Among these, from the viewpoint of enhancing the gas permeability of main body 3B, 4-methyl-1-pentene (co)polymers are preferred. In addition, from the viewpoint of enhancing the heat resistance of main body 3B, 4-methyl-1-pentene (co)polymers, polystyrene, polypropylene, and cyclic olefin (co)polymers are preferred.

The instantaneous value of the Shore D hardness of main body 3B is, for example, 60 to 90. The instantaneous value of Shore D hardness is a value obtained by contacting a push needle with a part of main body 3B using a durometer hardness tester (D type) and immediately reading the scale (in accordance with ASTM D2240). When the instantaneous value of the Shore D hardness is in the range of 60 to 90, transparent resin sheet 5B described below can be attached to main body 3B with a satisfactory appearance.

Frame 30B is composed of a rectangular frame-shaped member. Frame 30B constitutes the outer shape of culture vessel 1B.

Main body-side compartment 31B is provided in the space surrounded by frame 30B. Main body-side compartment 31B is provided integrally with frame 30B.

Main body-side compartment 31B includes a plurality of cylindrical portions 310B and bottom plate portion 311B.

Cylindrical portion 310B is cylindrical and opens in the vertical direction. In the present embodiment, the shape of the opening of cylindrical portion 310B (in other words, the outer shape in plan view) is circular. Cylindrical portions 310B are arranged in the left-right direction and the front-rear direction. The number of cylindrical portions 310B matches the number of compartments 10B for culturing cells. The number of cylindrical portions 310B may be, for example, 6, 24, 96, or 384. Of course, the number of cylindrical portions 310B may be other than 6, 24, 96, and 384.

Adjacent cylindrical portions 310B are connected by a connecting portion (not illustrated). In addition, the left end cylindrical portion 310B, the right end cylindrical portion 310B, the front end cylindrical portion 310B, and the rear end cylindrical portion 310B are connected to the inner peripheral surface of frame 30B via a connecting portion (not illustrated).

The space defined by the inner peripheral surface of cylindrical portion 310B (hereinafter referred to as "the inner space of cylindrical portion 310B") corresponds to an example of a through hole of the base member. The inner space of cylindrical portion 310B, together with transparent resin sheet 5B described below, constitutes compartment 10B for culturing cells.

Bottom plate portion 311B is a plate-shaped member that connects the lower ends of cylindrical portions 310B to each other, as illustrated in FIG. 10. The outer edge of bottom plate portion 311B is connected to the inner peripheral surface of frame 30B over the entire circumference. The lower surface of bottom plate portion 311B is also the lower surface of main body-side compartment 31B. In addition, the lower surface of bottom plate portion 311B is also the lower surface of main body 3B. Therefore, the lower surface of bottom plate portion 311B corresponds to an example of the lower surface of the base member.

Transparent resin sheet 5B is attached to the lower surface of main body 3B via pressure-sensitive adhesive layer 4B. Specifically, transparent resin sheet 5B is attached to the lower surface of bottom plate portion 311B in main body-side compartment 31B via pressure-sensitive adhesive layer 4B. As a result, transparent resin sheet 5B closes the bottom surface of cylindrical portion 310B and constitutes the bottom surface of vessel 1B.

Transparent resin sheet 5B is a sheet made of a transparent resin. Examples of the transparent resin include acrylic resins, cyclic olefin (co)polymers, 4-methyl-1-pentene (co)polymers, olefin resins including polypropylene and polyethylene, polyester resins including polyolefin terephthalate (PET), polystyrene resins, ethylene-vinyl alcohol (EVOH) resins, polyvinyl alcohol (PVOH) resins, and polyvinylidene chloride (PVDC).

From the viewpoint of enhancing the metabolic activity of cells by maintaining aerobic conditions inside compartment 10B, it is preferable that the oxygen permeability of transparent resin sheet 5B is 200 to 60,000 cm³/ (m² × 24hr × atm). From the viewpoint of effectively enhancing oxygen permeability, it is preferable that transparent resin sheet 5B is a sheet-shaped member containing a polymer having a structural unit derived from 4-methyl-1-pentene. Furthermore, according to the findings of the present inventors, polymers having structural units derived from 4-methyl-1-pentene have a lower adsorption amount of agents such as fluorescent dyes than conventionally used polydimethylsiloxane (PDMS). Therefore, when transparent resin sheet 5B is a sheet-shaped member containing a polymer having a structural unit derived from 4-methyl-1-pentene, it is less likely that the observation efficiency will decrease due to the adsorption of agents by transparent resin sheet 5B.

The above oxygen permeability is a value [cm³/ (m² × 24hr × atm)] measured using a differential pressure gas permeability measuring device MT-C3 manufactured by Toyo Seiki Seisaku-sho, Ltd., at a temperature of 23°C and a humidity of 0%. The measurement sample is prepared by cutting a 90 × 90 mm test piece from the transparent resin sheet 5, and the measurement section diameter is set to 70 mm (with transmission area of 38.46 cm²). When a high oxygen permeability is expected, it is more preferable to apply an aluminum mask to the measurement sample in advance to set the actual transmission area to 5.0 cm².

Pressure-sensitive adhesive layer 4B attaches transparent resin sheet 5B to the lower surface of bottom plate portion 311B in main body-side compartment 31B. Known pressure-sensitive adhesives such as acrylic, silicone, urethane, and rubber-based pressure-sensitive adhesives can be used for pressure-sensitive adhesive layer 4B. Among these, acrylic and silicone pressure-sensitive adhesives are preferable due to the ease of adjusting adhesive properties, and acrylic pressure-sensitive adhesives are more preferable.

FIG. 11 is a plan view of pressure-sensitive adhesive layer 4B. Pressure-sensitive adhesive layer 4B includes pressure-sensitive adhesive layer main body 40B and a plurality of openings 41B.

Pressure-sensitive adhesive layer main body 40B is a sheet-shaped member that is attached to the lower surface of main body-side compartment 31B (specifically, bottom plate portion 311B) and transparent resin sheet 5B.

Opening 41B is provided in pressure-sensitive adhesive layer main body 40B. Opening 41B extends through pressure-sensitive adhesive layer main body 40B in the vertical direction. In the state where the pressure-sensitive adhesive layer main body is attached to the lower surface of main body-side compartment 31B, opening 41B is disposed under through hole 310c of main body-side compartment 31B, as illustrated in FIG. 10. As illustrated in FIGS. 10 and 11, the outer shape of opening 41B is larger than the outer shape of through hole 310c in plan view. In FIG. 11, opening 41B is illustrated by a solid line. In FIG. 11, through hole 310c is illustrated by a two-dot chain line.

In the present embodiment, the outer shape of opening 41B in plan view is circular, as illustrated in FIG. 11. Hereinafter, when referring to the outer shape of opening 41B, it means the outer shape of opening 41B in plan view. The outer shape of through hole 310c is also circular. The outer shape of opening 41B is similar to the outer shape of through hole 310c. In the present embodiment, the center of the outer shape of opening 41B coincides with the center of the outer shape of through hole 310c. Therefore, the peripheral portion of opening 41B does not overlap with through hole 310c in plan view.

In the present embodiment, pressure-sensitive adhesive layer 4B is formed of a pressure-sensitive adhesive that satisfies the following conditions 1 and 2.

(Condition 1) The residual rate of Rhodamine B in the aqueous solution obtained by the following procedure is 20% or more: a test piece cut to a size of 10 mm × 10 mm is affixed to the bottom surface of a tissue culture-treated polystyrene (TCPS) well having an opening diameter of 16.2 mm, a well depth of 18 mm, and a well capacity of 3.5 mL; the well is filled with 0.5 mL of a phosphate-buffered saline (PBS) solution of Rhodamine B at a concentration of 10 µmol/L and left to stand at 23°C for 48 hours.

(Condition 2) To the test piece of the pressure-sensitive adhesive having a width of 5 mm, the generated load when a dynamic strain of 0.05% is applied using a solid viscoelasticity measuring device is 20g or less.

According to the findings of the present inventors, pressure-sensitive adhesives tend to adsorb agents such as fluorescent dyes, which can reduce the concentration of an agent introduced into compartment 10B. For this reason, the following is considered to occur: when attempting to examine the viability or activity of cells after culturing by using a conventional culture vessel with a pressure-sensitive adhesive, it is difficult to sufficiently detect signals from an agent used to detect a biologically-derived substance, making it difficult to improve observation efficiency. Similarly, agents that assist in cell culture may also be adsorbed, which may prevent the expected improvement in cell culture efficiency.

Based on the above findings, the inventors have confirmed the adsorption amounts of various agents for a plurality of pressure-sensitive adhesives and found that Rhodamine B can serve as an indicator for predicting the amounts of various agents adsorbed by pressure-sensitive adhesives. In other words, compared to other agents, Rhodamines are more likely to exhibit differences in adsorption amounts for each pressure-sensitive adhesive. Using a pressure-sensitive adhesive with a low adsorption amount of Rhodamine B allows for high observation efficiency and culture efficiency even when other agents are used for the observation, and also culture efficiency is sufficiently enhanced when cell culture is promoted by using other agents.

Condition 1 is based on these findings and specifies that the pressure-sensitive adhesive forming pressure-sensitive adhesive layer 4B has a low adsorption amount of Rhodamine B. A pressure-sensitive adhesive that satisfies condition 1 is less likely to adsorb various agents at an exposed site on the inner surface of compartment 10B. This enhances the signal detectability from agents in compartment 10B, thereby improving the observation efficiency of biologically-derived substances and the culture efficiency of cells by agents.

Regarding condition 1, in the present embodiment, the pressure-sensitive adhesive is cut into a size of 10 mm × 10 mm to create a test piece. The thickness of the test piece is the thickness of a purchased pressure-sensitive adhesive itself when it is a commercial product, and it is also the thickness of the pressure-sensitive adhesive which is used for pressure-sensitive adhesive layer 4B. The test piece is affixed to the bottom surface of a tissue culture-treated polystyrene well having an opening diameter of 16.2 mm, a well depth of 18 mm, and a well capacity of 3.5 mL. The well is filled with 0.5 mL of a phosphate-buffered saline (PBS) solution of Rhodamine B at a concentration of 5 µM and left to stand at 23°C for 48 hours. When the residual rate of Rhodamine B in the aqueous solution after standing is 20% or more (i.e., the concentration of Rhodamine B in the aqueous solution after standing is 1 µM or more), the pressure-sensitive adhesive forming pressure-sensitive adhesive layer 4B is considered to satisfy condition 1.

The amount of Rhodamine B in the aqueous solution after standing is measured by fluorescence analysis according to the following procedure.
Measuring device: FP-6600, manufactured by JASCO Corporation
Cell used: Quartz microcell
Bandwidth: Excitation side: 5nm, Fluorescence side: 6nm
Sensitivity (PMT voltage): 400V
Excitation wavelength: 555nm
Fluorescence measurement wavelength: 580nm
Scan speed: 2,000nm/min

From the above viewpoint, it is preferable that the residual rate of Rhodamine B in Condition 1 is 30% or more, and more preferably 40% or more. Although the upper limit of the residual rate of Rhodamine B in condition 1 is not particularly limited, the upper limit can be 100%.

Condition 2 means that the pressure-sensitive adhesive forming pressure-sensitive adhesive layer 4B is easily deformable even with a smaller force. Such a pressure-sensitive adhesive easily conforms to the shape of the bottom surface (lower surface) of main body-side compartment 31B and is less likely to cause fine gaps or bends during the attaching. Therefore, a pressure-sensitive adhesive that satisfies condition 2 is less likely to cause a decrease in observation efficiency due to bending transparent resin sheet 5B or causing unintended refraction of light at the peripheral portion of through hole 310c due to these gaps or bends. Furthermore, a pressure-sensitive adhesive that satisfies condition 2 reduces the contact area with a liquid (culture medium or the like) filling the inside of compartment 10B, thereby reducing the adsorption amount of various agents by pressure-sensitive adhesive layer 4B. These enhance the signal detectability from agents in compartment 10B, thereby improving the observation efficiency of biologically-derived substances.

Regarding condition 2, in the present embodiment, the pressure-sensitive adhesive is cut into a size of 5 mm in width and 30 mm in length to create a test piece. The thickness of the test piece is the thickness of the pressure-sensitive adhesive which is used for pressure-sensitive adhesive layer 4B. In the case of performing solid viscoelasticity measurement under the following conditions, when the generated load for applying a dynamic strain of 0.05% to the test piece under the following conditions of solid viscoelasticity measurement is 20g or less, the pressure-sensitive adhesive is considered to satisfy condition 2.
Measuring device: RSA-III, manufactured by TA Instruments Japan Inc.
Deformation mode: Tensile
Temperature: 23°C
Frequency: 1Hz
Strain: 0.05%
Atmospheric environment: Nitrogen atmosphere

From the above viewpoint, the generated load when a dynamic strain of 0.05% is applied in condition 2 is preferably 18g or less, more preferably 15g or less. Although the lower limit of the generated load when a dynamic strain of 0.05% is applied in condition 2 is not particularly limited, from the viewpoint of facilitating attachment, the lower limit can be 2g or more, preferably 5g or more.

It is preferable that pressure-sensitive adhesive layer 4B is formed by a pressure-sensitive adhesive that further satisfies the following conditions. It should be noted that the following conditions may be satisfied individually or in combination.

### (Condition 3)

The glass transition temperature (Tg) is -122°C or higher.

Condition 3 means that the pressure-sensitive adhesive forming pressure-sensitive adhesive layer 4B does not have excessive molecular mobility. In other words, when the mobility of the molecules that constitute a pressure-sensitive adhesive is high, the pressure-sensitive adhesive tends to adsorb agents more easily. In contrast, a pressure-sensitive adhesive that satisfies condition 3 has a moderately low mobility of the molecules that constitute the pressure-sensitive adhesive, and thus is less likely to adsorb various agents on an exposed site on the inner surface of compartment 10B. This enhances the signal detectability from agents in compartment 10B, thereby improving the observation efficiency of biologically-derived substances and the culture efficiency of cells by the agents. Particularly, when the pressure-sensitive adhesive is an acrylic pressure-sensitive adhesive, a higher Tg significantly reduces the amount of agent adsorption, thereby markedly increasing the observation efficiency of biologically-derived substances and the culture efficiency of cells.

Regarding condition 3, in the present embodiment, the solid viscoelastic temperature dispersion measurement of the pressure-sensitive adhesive is measured under the following conditions, and the peak top value of the loss tangent (tanδ) obtained is used as the glass transition temperature (Tg) of the pressure-sensitive adhesive.

### (Measurement Conditions)

Measuring device: RSA-III, manufactured by TA Instruments
Deformation mode: Tensile
Temperature range: -100°C to 25°C
Damping rate: 3°C/min
Frequency: 1Hz
Strain: 0.05%
Atmospheric environment: Air

For enhancing tackiness, a lower Tg of the pressure-sensitive adhesive is preferable. In contrast, from the viewpoint of improving the observation efficiency of biologically-derived substances based on the above findings, a higher Tg of the pressure-sensitive adhesive is preferable. From the viewpoint of achieving both of these, the Tg of the pressure-sensitive adhesive in condition 3 is preferably -121°C to -30°C, more preferably - 80°C to -30°C, and particularly preferably -35°C to -30°C.

### (Condition 4)

It is an acrylic pressure-sensitive adhesive having a structure derived from an aromatic ring-containing compound.

An acrylic pressure-sensitive adhesive having a structure derived from an aromatic ring-containing compound has an aromatic ring (benzene ring) that reduces molecular mobility. Therefore, a pressure-sensitive adhesive that satisfies condition 3 has a low mobility of the molecules that constitute the pressure-sensitive adhesive, and thus is less likely to adsorb various agents on an exposed site on the inner surface of compartment 10B. This enhances the signal detectability from agents in compartment 10B, thereby improving the observation efficiency of biologically-derived substances and the culture efficiency of cells by the agents.

Regarding condition 3, in the present embodiment, the surface of the pressure-sensitive adhesive is measured by Fourier Transform Infrared Spectroscopy (FT/IR) under the following conditions, and when a peak corresponding to the structure derived from an aromatic ring-containing compound at 700 cm⁻¹ is confirmed in the obtained IR spectrum, the pressure-sensitive adhesive is considered to satisfy condition 4.

### (Measurement Conditions)

Measuring device: Nicolet iS50, manufactured by Thermo Fisher Scientific
Measurement method: Total reflection measurement method using Ge prism (ATR method)
Resolution: 4 cm⁻¹
Number of accumulations: 32 times

### (Condition 5)

It includes a silicone pressure-sensitive adhesive, and the ratio ((Si-CH₃)/(Si-O)) between the peak intensity at 843 cm⁻¹ derived from Si-CH₃ and the peak intensity at 1068 cm⁻¹ derived from Si-O in the IR spectrum is 0.500 or more and 0.600 or less.

### (Condition 6)

It includes a silicone pressure-sensitive adhesive, and the ratio ((Si-CH₃)/(Si-O)) between the peak intensity at 756 cm⁻¹ derived from Si-CH₃ and the peak intensity at 1068 cm⁻¹ derived from Si-O in the IR spectrum is 0.280 or more and 0.300 or less.

Conditions 5 and 6 specify the amount of terminal methyl groups relative to the siloxane bonds in a silicone pressure-sensitive adhesive, indicating that the molecular weight of the silicone pressure-sensitive adhesive is of an appropriate size. A pressure-sensitive adhesive that satisfies condition 5 or condition 6 has a moderately low mobility of the molecules that constitute the pressure-sensitive adhesive, and thus is less likely to adsorb various agents on an exposed site on the inner surface of compartment 10B. This enhances the signal detectability from agents in compartment 10B, thereby improving the observation efficiency of biologically-derived substances and the culture efficiency of cells by the agents. On the other hand, in a pressure-sensitive adhesive that satisfies condition 5 or condition 6, the molecules that constitute the pressure-sensitive adhesive have sufficient mobility, and therefore the pressure-sensitive adhesive has sufficiently high tackiness.

A pressure-sensitive adhesive that satisfies condition 5 or condition 6 may satisfy either one or both of these conditions.

In addition, conditions 5 and 6 are the ratios of the respective peak intensities in the IR spectrum obtained under the same conditions as condition 3.

(Condition 7) The residual rate of Rhodamine 6G in the aqueous solution obtained by the following procedure is 20% or more: a test piece cut to a size of 10 mm × 10 mm is affixed to the bottom surface of a tissue culture-treated polystyrene well having an opening diameter of 16.2 mm, a well depth of 18 mm, and a well capacity of 3.5 mL, and the well is filled with 0.5 mL of a PBS solution of Rhodamine 6G at a concentration of 5 µM and left to stand at 23°C for 48 hours.

According to the findings of the inventors, in a similar manner to Rhodamine B in condition 1, Rhodamine 6G is also more likely to exhibit differences in adsorption amounts depending on the pressure-sensitive adhesive compared to other agents. A pressure-sensitive adhesive with a low adsorption amount of Rhodamine 6G tend to have low adsorption amounts for other agents as well. Therefore, a pressure-sensitive adhesive that satisfies condition 7 is less likely to adsorb various agents on an exposed site on the inner surface of compartment 10B. This enhances the signal detectability from agents in compartment 10B, thereby improving the observation efficiency of biologically-derived substances and the culture efficiency of cells by the agents.

Condition 7 is the residual rate of Rhodamine 6G in the aqueous solution after standing, obtained under the same conditions as condition 1, except that a 5 µM Rhodamine 6G aqueous solution is used instead of a 0.5 mL/well Rhodamine B aqueous solution.

From the above viewpoint, the residual rate of Rhodamine 6G in condition 1 is preferably 30% or more, more preferably 40% or more, and even more preferably 50% or more. Although the upper limit of the residual rate of Rhodamine 6G in condition 7 is not particularly limited, it can be 100%.

(Condition 8) The weight average molecular weight (Mw) is 1,000 or more and 5,000,000 or less.

Condition 8 means that the molecular weight of a pressure-sensitive adhesive is of an appropriate size. A pressure-sensitive adhesive that satisfies condition 8 has a moderately low mobility of the molecules that constitute the pressure-sensitive adhesive, and thus is less likely to adsorb various agents on an exposed site on the inner surface of compartment 10B. This enhances the signal detectability from agents in compartment 10B, thereby improving the observation efficiency of biologically-derived substances and the culture efficiency of cells by the agents. On the other hand, in a pressure-sensitive adhesive that satisfies condition 8, the molecules that constitute the pressure-sensitive adhesive have sufficient mobility, and therefore the pressure-sensitive adhesive has sufficiently high tackiness.

From the above viewpoint, the weight average molecular weight (Mw) of the pressure-sensitive adhesive in condition 8 is preferably 2,000 or more and 2,000,000 or less, more preferably 3,000 or more and 1,000,000 or less.

Condition 8 is the weight average molecular weight of the pressure-sensitive adhesive measured by gel permeation chromatography (GPC) under the following conditions.
Column: 2 × PLgel 5µ MIXED-C (7.5 mm × 300 mm), manufactured by Agilent Technologies
Column temperature: 40°C
Mobile phase: Tetrahydrofuran for HPLC (containing stabilizer), (manufactured by Fujifilm Wako Pure Chemical Corporation)
Flow rate: 1 mL/min
Injection volume: 100 µL
Detection: RI (Refractive Index)
Column calibration: Monodisperse polystyrene (EasiCal PS-1, manufactured by Agilent Technologies)
Molecular weight calibration: Relative calibration method (polystyrene equivalent)
Device: 515 HPLC Pump/717plus (auto-injector)/Shodex RI-101 (differential refractive index detector), manufactured by Nihon Waters K.K.

(Condition 9) The storage modulus (E') is 1 × 10⁵ Pa or more and 1 × 10¹⁰ Pa or less.

(Condition 10) The loss tangent (tanδ), which is the ratio (E"/ E') of the storage modulus (E') to the loss modulus (E"), is 0.0100 or more and 1 or less.

(Condition 11) The loss modulus (E") is 1 × 10⁶ Pa or more and 5 × 10⁸ Pa or less.

Regarding conditions 9, 10, and 11, in the present embodiment, the above values are obtained by the solid viscoelastic measurement performed under the same measurement conditions as condition 2.

The storage modulus (E') of the pressure-sensitive adhesive in condition 9 can be, for example, 1×10⁶ Pa or more, 1 × 10⁷ Pa or more, 1 × 10⁸ Pa or more, or 1 × 10⁹ Pa or more, and can also be 1 × 10⁹ Pa or less, 1 × 10⁸ Pa or less, 1 × 10⁷ Pa or less, or 1 × 10⁶ Pa or less.

The loss tangent (tanδ) of the pressure-sensitive adhesive in condition 10 can be, for example, 0.0500 or more, 0.1000 or more, 0.1500 or more, 0.2000 or more, 0.2500 or more, 0.3000 or more, 0.3500 or more, 0.4000 or more, 0.4500 or more, 0.5000 or more, 0.5500 or more, 0.6000 or more, 0.6500 or more, 0.7000 or more, 0.7500 or more, 0.8000 or more, 0.8500 or more, 0.9000 or more, or 0.9500 or more, and can also be 0.9500 or less, 0.9000 or less, 0.8500 or less, 0.8000 or less, 0.7500 or less, 0.7000 or less, 0.6500 or less, 0.6000 or less, 0.5500 or less, 0.5000 or less, 0.4500 or less, 0.4000 or less, 0.3500 or less, 0.3000 or less, 0.2500 or less, 0.2000 or less, 0.1500 or less, or 0.1000 or less.

The loss modulus (E") of a pressure-sensitive adhesive in condition 10 is preferably 1 × 10⁶ Pa or more and 5 × 10⁸ Pa or less, more preferably 5 × 10⁶ Pa or more and 1 × 10⁸ Pa or less. When the loss modulus (E") of the pressure-sensitive adhesive is within this range, the viscosity of the pressure-sensitive adhesive is low, so that an agent is less likely to remain inside the pressure-sensitive adhesive, resulting in better agent adsorption.

(Condition 11) The peel strength of a pressure-sensitive adhesive layer at -50°C is 0.1 N/25 mm or more.

The peel strength of the pressure-sensitive adhesive layer in condition 11 can be, for example, 0.2 N/25 mm or more, 0.3 N/25 mm or more, 1 N/25 mm or more, 5 N/25 mm or more, 10 N/25 mm or more, 15 N/25 mm or more, or 20 N/25 mm or more.

When the peel strength of a pressure-sensitive adhesive layer at -50°C is equal to or more than the lower limit, the structural body and the base member or sheet are firmly bonded to each other even at a low temperature, preventing delamination due to impact, or the like. In other words, impact resistance at a low temperature can be improved.

The upper limit of the peel strength of the pressure-sensitive adhesive layer at - 50°C is not particularly limited and can be, for example, 200 N/25 mm or less.

### (Other Descriptions regarding Pressure-Sensitive Adhesive Layer 4B)

Pressure-sensitive adhesive layer 4B may be formed by adhering a pressure-sensitive adhesive composition to both sides of a base layer (support), or by impregnating a base layer (support) such as a nonwoven fabric with a pressure-sensitive adhesive composition. Alternatively, pressure-sensitive adhesive layer 4B may be formed by a pressure-sensitive adhesive without a base layer (support), known as a base member-less adhesive. Among these, it is preferable to use a pressure-sensitive adhesive including a base layer since it is easy to form an opening and to attach the pressure-sensitive adhesive to the lower surface of bottom plate portion 311B in main body-side compartment 31B. In addition, in order to facilitate reducing the thickness of pressure-sensitive adhesive layer 4B, it is preferable that the pressure-sensitive adhesive is applied to both sides of the base layer (support).

The thickness of pressure-sensitive adhesive layer 4B is preferably 20 µm or more and 150 µm or less, more preferably 30 µm or more and 100 µm or less, and even more preferably 40 µm or more and 70 µm or less. The thicker the pressure-sensitive adhesive layer 4B, the easier it is to form an opening and attach the pressure-sensitive adhesive layer to the main body-side compartment. The thinner the pressure-sensitive adhesive layer 4B, the lower the adsorption amount of various agents by pressure-sensitive adhesive layer 4B, thereby enhancing the signal detectability from the agent in compartment 10B, and thereby improving the observation efficiency of biologically-derived substances and the culture efficiency of cells by the agent.

Pressure-sensitive adhesive layer 4B is preferably transparent from the viewpoint of enhancing the observation efficiency of biologically-derived substances. Specifically, the total light transmittance measured in accordance with JIS K 7361-1 is preferably 50% or more and 100% or less, more preferably 70% or more and 100% or less.

In the present embodiment, by using the above-mentioned pressure-sensitive adhesive layer 4B, it is possible to reduce the amount of an agent adsorbed by pressure-sensitive adhesive layer 4B and the bending of pressure-sensitive adhesive layer 4B, thereby preventing the decrease in observation efficiency of biologically-derived substances derived from pressure-sensitive adhesive layer 4B.

### [Third Embodiment]

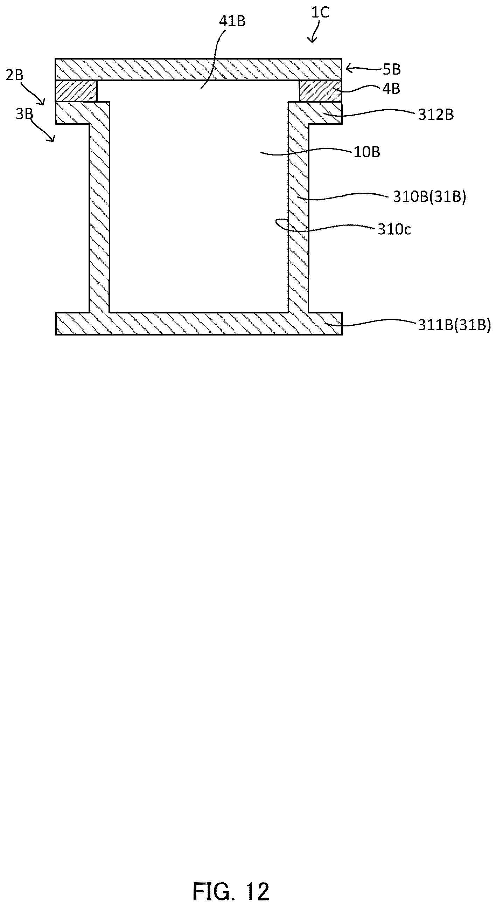

FIG. 12 is a partial cross-cross-sectional view of culture vessel 1C related to Embodiment 2. Culture vessel 1C differs from culture vessel 1B related to Embodiment 2 in the configuration of main body-side compartment 31B and the positions of pressure-sensitive adhesive layer 4B and transparent resin sheet 5B. Hereinafter, descriptions substantially the same as those of Embodiment 2 are omitted.

Main body-side compartment 31B includes a plurality of cylindrical portions 310B, bottom plate portion 311B, and upper plate portion 312B.

Cylindrical portion 310B is a recess whose lower portion is closed by bottom plate portion 311B and which opens only upward. The other configuration of cylindrical portions 310B is the same as in Embodiment 1.

Bottom plate portion 311B, as illustrated in FIG. 11, is a plate-shaped member that connects the lower ends of cylindrical portions 310B to each other. However, in the present embodiment, no opening is formed in bottom plate portion 311B, and therefore, bottom plate portion 311B forms the bottom surface of compartment 10B.

Upper plate portion 312B is a plate-shaped member that connects the upper ends of cylindrical portions 310B to each other, as illustrated in FIG. 11. The outer edge of upper plate portion 312B is connected to the inner peripheral surface of frame 30B over the entire circumference. The upper surface of upper plate portion 312B is also the upper surface of main body-side compartment 31B. Moreover, the lower surface of upper plate portion 312B is also the upper surface of main body 3B. Therefore, the upper surface of upper plate portion 312B corresponds to an example of the upper surface of the base member.

In the present embodiment, transparent resin sheet 5B is attached to the upper surface of main body 3B via pressure-sensitive adhesive layer 4B. Specifically, transparent resin sheet 5B is attached to the upper surface of upper plate portion 312B in main body-side compartment 31B via pressure-sensitive adhesive layer 4B. As a result, transparent resin sheet 5B closes the upper surface of vessel 1B and seals compartment 10B (the inner space of cylindrical portion 310B). In addition, pressure-sensitive adhesive layer 4B attaches transparent resin sheet 5B to the upper surface of upper plate portion 312B in main body-side compartment 31B.

In the present embodiment as well, pressure-sensitive adhesive layer 4B includes pressure-sensitive adhesive layer main body 40B and a plurality of openings 41B (see FIG. 11). The outer shape of opening 41B is larger than the outer shape of through hole 310c in plan view, as illustrated in FIG. 11. Moreover, the center of the outer shape of opening 41B coincides with the center of the outer shape of through hole 310c. Therefore, the peripheral portion of opening 41B does not overlap with through hole 310c in plan view.

In the present embodiment as well, a pressure-sensitive adhesive layer formed by the same pressure-sensitive adhesive as pressure-sensitive adhesive layer 4B in Embodiment 2 is used as pressure-sensitive adhesive layer 4B. This reduces the amount of an agent adsorbed by pressure-sensitive adhesive layer 4B and the bending of pressure-sensitive adhesive layer 4B, thereby preventing the decrease in observation efficiency of biologically-derived substances derived from pressure-sensitive adhesive layer 4B and the decrease in cell culture efficiency.

### [Other Embodiments]

It should be noted that each of the above-described embodiments is an example of the present invention, and the present invention is not limited to the above-described embodiments. It goes without saying that various other embodiments are possible within the scope of the idea of the present invention.

For example, while the above embodiments describe a culture vessel for culturing cells, the present invention is not limited thereto. As long as compartment 10B to which a biologically-derived substance (including cells or the like) is applied is formed by attaching another member to the base member via the pressure-sensitive adhesive layer, the present invention can also be applied to vessels for storing cells or for reacting or measuring a biologically-derived substance, such as flow path chips.

In Embodiments 2 and 3, opening 41B of pressure-sensitive adhesive layer 4B is larger than the outer shape of through hole 310c, but the opening may be the same size as the outer shape, and there is nothing to prevent opening 41B of pressure-sensitive adhesive layer 4B from being smaller than the outer shape of through hole 310c. However, by making opening 41B of pressure-sensitive adhesive layer 4B larger than the outer shape of through hole 310c, the effect of preventing agent adsorption by pressure-sensitive adhesive layer 4B is significantly enhanced.

Furthermore, in place of transparent resin sheet 5B, other non-transparent members may also be used.

### Examples

The present invention will be described in detail based on Examples, but the present invention is not limited to these Examples.

### 1. Preparation and Measurement of Pressure-sensitive adhesive

For commercially available industrial pressure-sensitive adhesives, the measurements of agent residual rate, solid viscoelasticity, infrared spectroscopy, molecular weight, and differential scanning calorimetry, and low-temperature peel test were conducted according to the following procedures to investigate various physical properties.

### 1-1. Agent Residual rate

Each pressure-sensitive adhesive was cut into a size of 10 mm × 10 mm to create test pieces. Each test piece was affixed to the bottom surface of a tissue culture-treated polystyrene well having an opening diameter of 16.2 mm, a well depth of 18 mm, and a well capacity of 3.5 mL. The well was filled with 0.5 mL of an agent aqueous solution and left to stand at 23°C for 48 hours. The amount of the agent in the aqueous solution after standing was measured using fluorescence analysis for Rhodamine B and LC/MS analysis for other samples, according to the following procedure. Then, the ratio of the amount of the agent in the aqueous solution after standing to the amount of the agent in the filled aqueous solution (before standing) was determined (agent amount after standing/agent amount before standing), and the determined value was used as the residual rate of the agent in each pressure-sensitive adhesive.

### (Measurement Conditions for Fluorescence Analysis)

Measuring device: FP-6600, manufactured by JASCO Corporation
Cell used: Quartz microcell
Bandwidth: Excitation side: 5 nm, Fluorescence side: 6 nm
Sensitivity (PMT voltage): 400 V
Excitation wavelength: 555 nm (Rhodamine B)
Fluorescence measurement wavelength: 580 nm (Rhodamine B)
Scan speed: 2,000 nm/min

### (Measurement Conditions for LC/MS)

Measuring device: TQ-S micro (water), manufactured by Acquity UPLC I-class system
Ionization method: Electrospray Ionization (ESI), positive and negative ion detection
Detection method: Selected Reaction Monitoring (SRM)

Furthermore, the following solutions were used for measuring the residual rate of each sample.
Rhodamine B: PBS solution of Rhodamine B at a concentration of 5 µM
Troglitazone: Dimethyl sulfoxide (DMSO) solution of Troglitazone at a concentration of 50 µM
Ticlopidine hydrochloride: DMSO solution of Ticlopidine hydrochloride at a concentration of 50 µM
Isoproterenol hydrochloride: DMSO solution of Isoproterenol hydrochloride at a concentration of 50 µM
Leflunomide: DMSO solution of Leflunomide at a concentration of 50 µM
Cyclosporin A: DMSO solution of Cyclosporin A at a concentration of 10 µM

### 1-2. Solid Viscoelasticity Measurement

Each pressure-sensitive adhesive was cut into a test piece of 5 mm width and 30 mm length. Using a solid dynamic viscoelasticity measuring device, a dynamic strain of 0.05% was applied to each test piece under the following conditions, and the generated load was measured to determine the 0.05% strain load for each pressure-sensitive adhesive. Furthermore, from the measurement results, the storage modulus (E'), loss modulus (E"), and the loss tangent (tanδ), which is the ratio (E"/E') of the loss modulus to the storage modulus), were determined.
Measuring device: RSA-III, manufactured by TA Instruments
Deformation mode: Tensile
Temperature: 23°C
Frequency: 1Hz
Strain: 0.05%
Atmospheric environment: Nitrogen atmosphere

In addition, a test piece of each pressure-sensitive adhesive was subjected to solid viscoelastic temperature dispersion measurement using a solid dynamic viscoelasticity measuring device, and the peak top value of the obtained loss modulus was used as the glass transition temperature (Tg) of the pressure-sensitive adhesive.

### (Measurement Conditions)

Measuring device: RSA-III, manufactured by TA Instruments
Deformation mode: Tensile
Temperature range: -100°C to 25°C
Damping rate: 3°C/min
Frequency: 1Hz
Strain: 0.05%
Atmospheric environment: Air

### 1-3. Infrared Spectroscopy Measurement

The surface of each pressure-sensitive adhesive was measured under the following conditions using Fourier Transform Infrared Spectroscopy (FT/IR) to obtain the IR spectrum. For acrylic-based pressure-sensitive adhesives, when a peak corresponding to a styrene-derived structure was confirmed in the obtained IR spectrum, the pressure-sensitive adhesive was determined to have a styrene-derived structure. For silicone-based pressure-sensitive adhesives, in the IR spectrum, the ratio of the peak intensity at 843 cm⁻¹ derived from Si-CH₃ to the peak intensity at 1068 cm⁻¹ derived from Si-O (that is, (Si-CH₃)/(Si-O)), and the ratio of the peak intensity at 756 cm⁻¹ derived from Si-CH₃ to the peak intensity at 1068 cm⁻¹ derived from Si-O (that is, (Si-CH₃)/(Si-O)) were determined, and these were used as the 843/1068 ratio and 756/1068 ratio for each adhesive.

### (Measurement Conditions)

Measuring device: Nicolet iS50, manufactured by Thermo Fisher Scientific
Measurement method: Total reflection measurement method using Ge prism (ATR method)
Resolution: 4 cm⁻¹
Number of integrations: 32 times

### 1-4. Molecular Weight Measurement

The molecular weight of each pressure-sensitive adhesive was measured by Gel Permeation Chromatography (GPC) under the following conditions.
Column: 2 × PLgel 5µ MIXED-C (7.5 mm × 300 mm), manufactured by Agilent Technologies
Column temperature: 40°C
Mobile phase: Tetrahydrofuran for HPLC (containing stabilizer), (manufactured by Fujifilm Wako Pure Chemical Corporation)
Flow rate: 1 mL/min. Injection volume: 100 µL
Detection: RI (Refractive Index)
Column calibration: Monodisperse polystyrene (EasiCal PS-1, manufactured by Agilent Technologies)
Molecular weight calibration: Relative calibration method (polystyrene equivalent)
Device: 515 HPLC Pump/717plus (auto-injector)/Shodex RI-101 (differential refractive index detector), manufactured by Nihon Waters K.K.

### 1-5. Low-Temperature Peel Test

The peel strength of the pressure-sensitive adhesive layer at -50°C was measured according to the following method in accordance with JIS Z 0237.

In an environment of 20°C and 50 % RH, a film made of 4-methyl-1-pentene copolymer (TPX, manufactured by Mitsui Chemicals, Inc.) with a thickness of 50 µm was fixed to a SUS plate via each pressure-sensitive adhesive to prepare a test piece. The prepared test piece was stored at a temperature of -40°C to -50°C for 24 hours. Immediately after the storage, the test piece was used to measure peel strength in an atmosphere at -50°C by peeling the film against a SUS plate at 180° (180° peel method). After peeling approximately 10cm, the initial and final 15% were excluded and the average value was calculated, which was used as the peel strength value of the pressure-sensitive adhesive layer at -50°C.

### 2. Measurement Results

Table 1 shows the residual rates of agents in each pressure-sensitive adhesive. Table 2 shows other physical properties of each pressure-sensitive adhesive. Table 3 shows the results of the low-temperature peel test.

**[Table 1]**

| Pressure-sensitive adhesive No. | Material | Residual rate (%) | | | | | |
|---|---|---|---|---|---|---|---|
| | | Rhodamine B | Troglitazone | Ticlopidine hydrochloride | Isoproterenol hydrochloride | Leflunomide | Cyclosporin A |
| 1 | Acrylic-based | 26 | 69 | 63 | 79 | 70 | 70 |
| 2 | Acrylic-based | 49 | 77 | 65 | 78 | 72 | 71 |
| 3 | Acrylic-based | 5 | 73 | 72 | 79 | 73 | 76 |
| 4 | Acrylic-based | 49 | 77 | 76 | 80 | 76 | 73 |
| 5 | Acrylic-based | 43 | 101 | 75 | 80 | 76 | 71 |
| 6 | Acrylic-based | 60 | 61 | 66 | 71 | 66 | 52 |
| 7 | Acrylic-based | 38 | 68 | 73 | 72 | 72 | 74 |
| 8 | Silicone-based | 63 | 72 | 80 | 80 | 77 | 73 |
| 9 | Silicone-based | 18 | 80 | 80 | 79 | 77 | 79 |
| 10 | Silicone-based | 62 | 76 | 78 | 80 | 79 | 75 |

**[Table 2]**

| Pressure-sensitive adhesive No. | Material | Thickness (µm) | Base layer Type | Solid viscoelasticity measurement | | FT-IR measurement | | | Molecular weight (Mw) | DSC | | |
|---|---|---|---|---|---|---|---|---|---|---|---|---|
| | | | | 0.05% Strain load (g) | Tg (°C) | Styrene structure | 843/1068 ratio | 756/1068 ratio | | Storage modulus (E') (Pa) | Loss modulus (E"") (Pa) | Loss tangent (tanδ) (Pa) |
| 1 | Acrylic-based | 120 | Nonwoven Fabric | 12.2 | -38 | None | - | - | | 4.32 × 108 | 5.61 × 107 | 0.1298 |
| 2 | Acrylic-based | 90 | Nonwoven Fabric | 17.3 | -37 | None | - | - | | 7.73 × 108 | 9.46 × 107 | 0.1224 |
| 3 | Acrylic-based | 80 | Polyester | 19.4 | -24 | None | - | - | | 8.44 × 108 | 1.44 × 108 | 0.1701 |
| 4 | Acrylic-based | 50 | Polyester | 14.4 | -30 | Yes | - | - | | 1.12 × 109 | 9.77 × 107 | 0.0872 |
| 5 | Acrylic-based | 30 | Polyester | 4.8 | -32 | None | - | - | | 6.53 × 108 | 2.72 × 107 | 0.0416 |
| 6 | Acrylic-based | 50 | Polyethylene | 12.7 | -40 | None | - | - | | 9.57 × 108 | 5.23 × 107 | 0.0546 |
| 7 | Acrylic-based | 80 | Polyethylene | 13.7 | -26 | None | - | - | | 6.96 × 108 | 5.42 × 107 | 0.0779 |
| 8 | Silicone-based | 50 | None | 2.5 | -121 | - | 0.529 | 0.293 | 5190 | 6.96 × 106 | 5.55 × 106 | 0.7972 |
| 9 | Silicone-based | 95 | Polyethylene | 28.4 | -123 | - | 0.453 | 0.270 | 39100 | 1.27 × 109 | 4.14 × 107 | 0.0326 |
| 10 | Silicone-based | 40 | Polyester | - | -124 | - | 0.529 | 0.192 | 4730 | - | - | - |

**[Table 3]**

| Pressure-sensitive adhesive | Low-temperature peel strength (N/25mm) |
|---|---|
| Pressure-sensitive adhesive 4 | 0.39 |
| Pressure-sensitive adhesive 6 | 0.37 |
| Pressure-sensitive adhesive 9 | 8.18 |
| Pressure-sensitive adhesive 10 | 25.52 |

### 3. Observation Test

Culture vessel 1B as illustrated in FIGS. 9 and 10 was prepared. A 24-well plate frame with an open bottom surface of the wells was prepared by injection molding by using Toyo Styrene's Toyo Styrol G-200C as polystyrene, and pressure-sensitive adhesive 3B, pressure-sensitive adhesive 4B, or pressure-sensitive adhesive 9 was used as the pressure-sensitive adhesive to attach transparent resin sheet 5B made of 4-methyl-1-pentene copolymer (TPX, manufactured by Mitsui Chemicals, Inc.) to the frame.

To the inner surface of compartment 10B of culture vessel 1B, 0.5 mL of a 1.5mg/mL collagen coat solution was added, and then the excess collagen coat solution was removed. After standing at room temperature for 30 to 60 minutes, it was washed with Dulbecco's PBS (-) and dried overnight at room temperature.

Next, a culture medium containing primary frozen rat hepatocytes (0.5 mL) was seeded onto the culture surface of compartment 10B using a micropipette, covered with a polystyrene lid, and placed in an incubator to start culturing at 37°C under 5% CO₂. After 5 days of culture, a PBS solution of Rhodamine B at a concentration of 5 µM was introduced into compartment 10B to stain the cells.

After staining, the cells were observed using a phase contrast fluorescence microscope to confirm whether the cultured cells were sufficiently stained and clearly visible, and whether any decrease in visibility due to the bending of the pressure-sensitive adhesive (such as blurring at the peripheral portion) was observed.

**[Table 4]**

| Pressure-sensitive adhesive | Staining property | Visibility |
|---|---|---|
| Pressure-sensitive adhesive 3 | Staining of cells is insufficient and unclear | Decrease in visibility due to bending of pressure-sensitive adhesive is not observed |
| Pressure-sensitive adhesive 4 | Staining of cells is sufficient and clearly visible | Decrease in visibility due to bending of pressure-sensitive adhesive is not observed |
| Pressure-sensitive adhesive 9 | Staining of cells is insufficient and extremely unclear | Decrease in visibility due to bending of pressure-sensitive adhesive is also observed |

The results shown in Table 4 show that there are differences in the amount of the agent adsorbed by different pressure-sensitive adhesives. Furthermore, it is understood that pressure-sensitive adhesives with low adsorption of Rhodamine B (high residual rate) also have low adsorption of other agents (high residual rate).

From the results illustrated in Tables 1 to 4, it is understood that using a pressure-sensitive adhesive with low adsorption of Rhodamine B and a generated load of 20g or less when a dynamic strain of 0.05% is applied to a 5 mm wide test piece by using a solid viscoelasticity measuring device can improve the observation efficiency of cells (specifically, the biologically-derived substances reacted with each agent in Table 4).

### (Additional Note)

In implementing culture vessel 1 of Embodiment 1 described above, a gas-blocking sheet (not illustrated) that blocks the transmission of gas (specifically, oxygen) may be provided on the lower surface of gas-permeable sheet 5. Culture vessel 1 with such a configuration can switch between a state where air is not supplied to compartment 10 through gas-permeable sheet 5 (hereinafter referred to as "the first state of culture vessel 1") and a state where air is supplied to compartment 10 through gas-permeable sheet 5 (hereinafter referred to as "the second state of culture vessel 1").

The first state of culture vessel 1 corresponds to a state where a gas-blocking sheet is provided on the lower surface of gas-permeable sheet 5. The second state of culture vessel 1 corresponds to a state where the gas-blocking sheet is removed from the lower surface of gas-permeable sheet 5.

### [Configuration Example of Structural body Related to Second Aspect]

According to the second aspect of the present invention, the following structural body is provided.

A structural body including:
a base member including a through hole or a recess; and
a pressure-sensitive adhesive layer provided on one surface of the base member, in which
a vessel that allows observation of a biologically-derived substance is formed by adhering another base member or a sheet via the pressure-sensitive adhesive layer to close at least one surface of the through hole or close the recess, and
a pressure-sensitive adhesive forming the pressure-sensitive adhesive layer has the following features:
   a residual rate of Rhodamine B in a solution is 20% or more, the solution being obtained by affixing a test piece of the pressure-sensitive adhesive cut to a size of 10 mm × 10 mm to a bottom surface of a tissue culture-treated polystyrene well having an opening diameter of 16.2 mm, a well depth of 18 mm, and a well capacity of 3.5 mL, filling the well with 0.5 mL of a phosphate-buffered saline solution of the Rhodamine B at a concentration of 5 µM, and allowing the phosphate-buffered saline solution to stand at 23°C for 48 hours, and
   a generated load when a dynamic strain of 0.05% is applied to the test piece of the pressure-sensitive adhesive by using a solid viscoelasticity measuring device is 20g or less, the test piece having a width of 5 mm.

In addition, according to the second aspect of the present invention, in the structural body,
the pressure-sensitive adhesive has the residual rate of the Rhodamine B of 30% or more.

In addition, according to the second aspect of the present invention, in the structural body,
the pressure-sensitive adhesive layer is a layer formed by a pressure-sensitive adhesive having a glass transition temperature (Tg) of -122°C or higher.

In addition, according to the second aspect of the present invention, in the structural body,
the pressure-sensitive adhesive layer is a layer formed by an acrylic pressure-sensitive adhesive or a silicone pressure-sensitive adhesive.

In addition, according to the second aspect of the present invention, in the structural body,
the pressure-sensitive adhesive layer is a layer formed by an acrylic pressure-sensitive adhesive having a styrene-derived structure.

In addition, according to the second aspect of the present invention, in the structural body,
the pressure-sensitive adhesive layer is a layer formed by a silicone pressure-sensitive adhesive with a ratio ((Si-CH₃)/(Si-O)) of 0.500 or more and 0.600 or less between a peak intensity at 843 cm⁻¹ derived from Si-CH₃ and a peak intensity at 1068 cm⁻¹ derived from Si-O in an IR spectrum.

In addition, according to the second aspect of the present invention, in the structural body,
the pressure-sensitive adhesive layer is a layer formed by a silicone pressure-sensitive adhesive with a ratio ((Si-CH₃)/(Si-O)) of 0.280 or more and 0.300 or less between a peak intensity at 756 cm⁻¹ derived from Si-CH₃ and a peak intensity at 1068 cm⁻¹ derived from Si-O in the IR spectrum.

In addition, according to the second aspect of the present invention, in the structural body,
peel strength of the attachment layer at -50°C is 0.1 N/25 mm or more, the peel strength being measured by a method in accordance with JIS Z 0237.

In addition, according to the second aspect of the present invention, in the structural body,
the pressure-sensitive adhesive layer includes a base layer and a pressure-sensitive adhesive composition adhering to or saturating the base layer.

In addition, according to the second aspect of the present invention, in the structural body,
the pressure-sensitive adhesive layer has a thickness of 20 µm or more and 150 µm or less.

In addition, according to the second aspect of the present invention, in the structural body,
the base member includes a plurality of through holes; and
the pressure-sensitive adhesive layer includes, under the through hole, an opening whose outer shape is larger than an outer shape of the through hole in plan view.

In addition, according to the second aspect of the present invention, a vessel that allows observation of a biologically-derived substance is provided, the vessel including:
the structural body according to the second aspect of the present invention as described above; and
another base member or a sheet attached to the structural body via the pressure-sensitive adhesive layer.

In addition, according to the second aspect of the present invention, in the vessel, the other base member or the sheet is a sheet with oxygen permeability.

In addition, according to the second aspect of the present invention, in the vessel,
the sheet with oxygen permeability contains a polymer having a structural unit derived from 4-methyl-1-pentene.

In addition, according to the second aspect of the present invention, in the vessel,
the other base member or the sheet forms a bottom surface of the vessel.

In addition, according to the second aspect of the present invention, a vessel that is a cell culture vessel is provided.

In addition, according to the first and second aspects of the present invention, the following structural body is provided:
a structural body constituting a culture vessel including a plurality of compartments for culturing a cell, the structural body including:
a base member including a through hole opening in a vertical direction, the through hole including a plurality of through holes; and
an attachment layer provided on a lower surface of the base member, the attachment layer attaching a sheet for closing the through hole to the lower surface, in which
peel strength of the attachment layer at -50°C is 0.1 N/25 mm or more, the peel strength being measured by a method in accordance with JIS Z 0237.

A structural body having peel strength of the attachment layer at -50°C within the above range has high impact resistance at a low temperature. In other words, even at a low temperature, the structural body and the sheet are firmly bonded, preventing peeling due to impact, or the like.

This application is entitled to and claims the benefits of Japanese Patent Application No. 2023-26893 and Japanese Patent Application No. 2023-26897 both dated February 24, 2023, the disclosures of which each including the specification, drawings and abstract are incorporated herein by reference in their entirety.

### Industrial Applicability

The culture vessel according to the present invention can be applied to the culture of various cells.

### Reference Signs List

1, 1B, 1C Culture vessel
10, 10B Compartment
2, 2B Structural body
3, 3B Main body
30, 30B Frame
300 Lower frame
300a Notch
301 Upper frame
31, 31B Main body-side compartment
310, 310B Cylindrical portion
310a, 310b, 310c Through hole
311, 311B Bottom plate portion
312 Upper plate portion
4, 4a, 4B Adhesive layer
40, 40a, 40B Adhesive layer main body
41, 41a, 41B Opening
420 Base layer
420a Base layer side main body
420b Base layer side opening
421 Upper adhesive layer
421a Upper layer side main body
421b Upper layer side opening
422 Lower adhesive layer
422a Lower layer side main body
422b Lower layer side opening
5, 5B Gas-permeable sheet
6a Upper protective film
6b Lower protective film

## Claims

1. A structural body constituting a culture vessel that includes a plurality of compartments for culturing cells, the structural body comprising:
a base member including a plurality of through holes each opening in a vertical direction; and
an attachment layer provided on a lower surface of the base member, the attachment layer attaching a sheet for closing the through hole to the lower surface, wherein
the attachment layer includes, under the through hole, an opening whose outer shape is larger than an outer shape of the through hole in a plan view.

2. The structural body according to claim 1, wherein a peripheral portion of the opening does not overlap with the through hole in the plan view.

3. The structural body according to claim 1, wherein:
the outer shape of the through hole and the outer shape of the opening are circular or regular polygonal; and
a difference between a radius of the opening and a radius of the through hole is smaller than half a distance between ends of adjacent openings among a plurality of the openings.

4. The structural body according to claim 3, wherein
a distance between a center of the opening and a center of the through hole is equal to or smaller than half the difference between the radius of the opening and the radius of the through hole.

5. The structural body according to claim 1, wherein
the outer shape of the through hole and the opening are circular, rectangular, or regular polygonal.

6. The structural body according to claim 1, wherein
the outer shape of the opening is similar to the outer shape of the through hole.

7. The structural body according to claim 1, wherein
the attachment layer is made of a pressure-sensitive adhesive.

8. The structural body according to claim 7, wherein
the attachment layer includes
a base layer,
a first adhesive layer provided on an upper surface of the base layer, and
a second adhesive layer provided on a lower surface of the base layer.

9. The structural body according to claim 7, wherein
the attachment layer includes
a base layer, and
a pressure-sensitive adhesive composition adhering to or saturating the base layer.

10. The structural body according to claim 7, wherein
the attachment layer is formed by the pressure-sensitive adhesive having a feature such that a residual rate of Rhodamine B in a solution is 20% or more, the solution being obtained by affixing a test piece of the pressure-sensitive adhesive cut to a size of 10 mm × 10 mm to a bottom surface of a tissue culture-treated polystyrene well having an opening diameter of 16.2 mm, a well depth of 18 mm, and a well capacity of 3.5 mL, filling the well with 0.5 mL of a phosphate-buffered saline solution of the Rhodamine B at a concentration of 5 µM, and allowing the phosphate-buffered saline solution to stand at 23°C for 48 hours.

11. The structural body according to claim 7, wherein
the attachment layer is formed by the pressure-sensitive adhesive having a glass transition temperature (Tg) of -122°C or higher.

12. The structural body according to claim 7, wherein
a thickness dimension H of the attachment layer satisfies 20 µm ≤ H ≤ 150 µm.

13. The structural body according to claim 1, wherein
peel strength of the attachment layer at -50°C is 0.1 N/25 mm or more, the peel strength being measured by a method in accordance with JIS Z 0237.

14. A vessel, comprising:
the structural body according to any one of claims 1 to 13; and
the sheet closing the through hole and, together with the through hole, forming a compartment of the plurality of compartments.

15. The vessel according to claim 14, wherein oxygen permeability of the sheet at a relative humidity of 0 % RH and a temperature of 23°C is 4,500 cm³/(m² × 24hr × atm) or more.

16. The vessel according to claim 14, wherein the sheet contains a 4-methyl-1-pentene polymer.

17. A structural body, comprising:
a base member including a through hole or a recess; and
a pressure-sensitive adhesive layer provided on one surface of the base member,
wherein
the structural body forms a vessel by adhering another base member or a sheet via the pressure-sensitive adhesive layer to close at least one surface of the through hole or close the recess, the vessel allowing observation of a biologically-derived substance, and
a pressure-sensitive adhesive forming the pressure-sensitive adhesive layer has features below
a residual rate of Rhodamine B in a solution is 20% or more, the solution being obtained by affixing a test piece of the pressure-sensitive adhesive cut to a size of 10 mm × 10 mm to a bottom surface of a tissue culture-treated polystyrene well having an opening diameter of 16.2 mm, a well depth of 18 mm, and a well capacity of 3.5 mL, filling the well with 0.5 mL of a phosphate-buffered saline solution of the Rhodamine B at a concentration of 5 µM, and allowing the phosphate-buffered saline solution to stand at 23°C for 48 hours, and
a generated load when a dynamic strain of 0.05% is applied to the test piece of the pressure-sensitive adhesive by using a solid viscoelasticity measuring device is 20g or less, the test piece having a width of 5 mm.

18. The structural body according to claim 17, wherein
the pressure-sensitive adhesive layer is a layer formed by an acrylic pressure-sensitive adhesive having a structure derived from an aromatic ring-containing compound.

19. The structural body according to claim 17, wherein
the pressure-sensitive adhesive layer is a layer formed by a silicone pressure-sensitive adhesive with a ratio ((Si-CH₃)/(Si-O)) of 0.500 or more and 0.600 or less between a peak intensity at 843 cm⁻¹ derived from Si-CH₃ and a peak intensity at 1068 cm⁻¹ derived from Si-O in an IR spectrum.

20. A vessel that allows observation of a biologically-derived substance, the vessel comprising:
the structural body according to any one of claims 17 to 19; and
another base member or a sheet attached to the structural body via the pressure-sensitive adhesive layer.
